# EUROPEAN PATENT APPLICATION

(11) **EP 4 461 230 A1**
(43) Date of publication of application: **13.11.2024**
(21) Application number: 23171969.1
(22) Date of filing: 07.05.2023
(51) Int. Cl.: A61B 8/08, A61B 8/00

(54) **ULTRASOUND SYSTEM**

(71) Applicant: Pulsify Medical, 3001 Leuven (BE)
(72) Inventor: MORAES, Rodrigo, 3000 Leuven (BE); STOFFELS, Steve, 3053 Haasrode (BE); BARBOSA, Daniel, 4710-089 Braga (PT)
(74) Representative: DTS Patent- und Rechtsanwälte PartmbB

(57) **Abstract**

The invention relates to an ultrasound system (1), in particular for cardiac monitoring, comprising a transducer array (3) with a plurality of transducer array elements (3a), a control unit (40), which is coupled to the transducer array (3), for controlling the transducer array (3), and a data processing unit (41) for receiving data from the transducer array (3) and processing the received data, wherein the system (1) further comprises a plurality of sub-arrays (4) defined as a collection of one or more transducer array elements (3a) and controlled as functional units for sending ultrasound pulses and/or receiving ultrasound signals, and at least one scan sequence module (42) configured for performing a scan sequence step, wherein the scan sequence module is configured to activate a sequence of sub-arrays (4), during the scan sequence step, for gathering ultrasound data. Additionally, the invention also relates to a method for ultrasound imaging of a body part of a subject.

## Description

The present invention relates to an ultrasound system, in particular for cardiac monitoring.

Wearable ultrasound monitoring systems are configured to monitor cardiac function over time. The ultrasound monitoring system can be body worn in a patch type format.

Once the ultrasound monitoring system, e.g., the ultrasound patch, has been applied to the body it is not yet "aware" of the features present inside the subject body. In particular, after scanning, well-trained operators are needed for detecting and discerning such features in order to proceed further with the gained information. Accordingly, any other user, e.g., a non-trained operator, may either not be able to proceed further after scanning, or possibly induce a misinterpretation concerning the scanned features inside the subject body.

Further, another problem may be that the user might be a, e.g., the above-mentioned, non-trained operator and, accordingly, it is also not ensured that the wearable ultrasound monitor system has been properly applied to the subject body, e.g., placed on the subject body.

A measurement needs to establish, which features are present within the field of view of the sensor of the system. At the highest level this may consist of two steps: firstly, performing a pulse-echo measurement inside the field of view of the sensor, i.e., executing a scan sequence, and, secondly, analyzing the measurement data to detect the features of interest.

When performing said scan sequence, in principle the topology or physiological features inside the field of view of the sensor are still unknown. Therefore, a more detailed scanning may be needed to establish a good 3D structure of all the features present inside the field of view of the sensor. However, a detailed 3D scan usually requires a lot of resources, when performed, (i.e., long acquisition time, high power consumption, bandwidth, memory, etc.) and would accordingly not be energy efficient.

Hence, there is a need for an ultrasound system and a method for performing the scan sequence in an efficient manner, while still allowing the relevant physiological regions and/or features of interest to be detected and identified. Further, there is a need for a feedback mechanism about the scanned features as well as about the positional correctness of the patch placement.

It is the goal of the present invention to provide an ultrasound system and a method, which satisfy the above-mentioned need and are accordingly able to perform a corresponding scan sequence in an efficient manner and to perform a corresponding analysis of features inside the subject body to provide such a feedback mechanism.

This problem is solved by an ultrasound system, in particular for cardiac monitoring, according to claim 1 of the attached set of claims. Further advantageous embodiments are given in the dependent claims.

The ultrasound system, in particular for cardiac monitoring, comprises:
- a transducer array with a plurality of transducer array elements,
- a control unit, which is coupled to the transducer array, for controlling the transducer array, and
- a data processing unit for receiving data from the transducer array and processing the received data.

The ultrasound system further comprises:
- a plurality of sub-arrays defined as a collection of one or more transducer array elements and controlled as functional units for sending ultrasound pulses and/or receiving ultrasound signals, and
- at least one scan sequence module configured for performing a scan sequence step, wherein the scan sequence module is configured to activate a sequence of sub-arrays, during the scan sequence step, for gathering ultrasound data.

The invention is based on the basic idea that, due to activating (e.g., only) a (particular) sequence of sub-arrays being defined by one or more transducer array elements, ultrasound data may be gathered in a more goal-oriented/target-oriented manner, i.e., only the most relevant data may be gathered, thereby allowing the ultrasound system to be operated in a more economical manner, in particular with respect to power consumption, as well as allowing the accuracy of the obtained ultrasound images to be increased. In particular, since each activation of a sub-array has a cost in power, the minimization of the number of sub-array positions and measurements per sub-array position, which are needed during the performance of the scan sequence, may result in said more economical operation of the ultrasound system (while maintaining good image quality). In other words, due to the scan sequence module performing said scan sequence, the ultrasound system may provide a more efficient scanning of a region of interest in the subject body and/or provide a good distribution of ultrasound measurements over said region of interest with a minimal amount of ultrasound shots/measurements. In particular, the sub-arrays are electronically defined and are not regarded as physical components.

Additionally, or alternatively, the system can further comprise:
- at least one reference module configured for obtaining and/or providing reference ultrasound data, and
- at least one feature detection module configured for interfacing with the reference module and for performing a determination step of the transducer array data to detect at least one feature of interest.

The feature detection module is configured to provide at least one detection information to the reference module determined by the determination step.

The detection of information in the sense of this disclosure is especially (but not limited to) detection of features of anatomical structures such as structures of organs or blood vessels, which may be identified from the obtained data from the transducer array. Such data may be especially ultrasound data.

The reference ultrasound data may comprise physiological and pathophysiological data. They may be obtained from the same patient or from other patients. In particular, the reference ultrasound data may be stored in a reference library. Such reference library can be embodied as a part of the system or as a separate system, to which the reference module is connected or connectable.

In particular, the invention can be based on the basic idea that, by performing an analysis of features inside the sensor's field of view, i.e., a determination step of the transducer array data, with a/the feature detection module, detection information may be generated for transmission to the reference module, to thereby provide the reference module with a basis, on which the reference module may further process the transducer array data in a selective and well-directed manner in dependence of the detected features of interest. Hence, due to the detection information obtained by the feature detection module, features of interest, in particular organ features, may be identified and discerned as well as it may be evaluated if the targeted features of interest are visible in the sensor's field of view. Accordingly, feedback (e.g., user feedback) regarding said features of interest themselves as well as regarding the correctness of the transducer array placement may be provided. In other words, a feature detection module is provided which may perform a determination step of the transducer array data, which represent features inside the transducer array's field of view, wherein the determination step may help to identify organ features, but also evaluate if the targeted organ features are visible in the field of view, thus being able to provide critical (user) feedback on the correctness of the patch placement.

In this feature detection module, the detection of the feature of interest, e.g., the left ventricle (LV) within a cardiac scan, may be a key step, which may serve not only to optimize and personalize the scanning process of the ultrasound system, but may also enable automated analysis of the feature's size and function.

Further, the feature detection module may facilitate a more focused ultrasound monitoring on a feature of interest instead of constant monitoring of an unrestricted area by the whole transducer array, e.g., in an automated manner. Accordingly, a power saving may be, e.g., automatically, enabled. The use of the feature detection module, i.e., the use of the detection information gained by the feature detection module, may enable to restrict monitoring on the region of interest of the feature of interest such as an organ, fluid carrying structure or other volumetric feature in the body, e.g. the heart, or a part of a target organ, in particular a ventricle or atrium, which during a cardiac cycle or selected phases of a cardiac cycle provides the required physiological and/or pathological information. For instance, the volume of the left ventricle instead of the volume of the total heart may be monitored to determine how much volume of blood the heart is pumping, in particular per cardiac cycle or a selected phase of a cardiac cycle. Overall, through this (e.g., automatic) restriction of monitoring, a less power consuming monitoring and a longer lifetime of the battery of the system and/or less frequent charging may be enabled. In other words, in addition to the items pointed out further above, the system may advantageously require less computational capacities and the power consumption of the ultrasound system may be reduced.

The system may be configured for cardiac monitoring, which may for example comprise monitoring of cardiac activity and/or cardiac properties of a subject, e.g., a patient. Monitoring cardiac activity may comprise monitoring of parameters such as cardiac rhythm and/or cardiac output.

The system has at least one transducer array, i.e., a device or unit comprising a plurality of transducer array elements. In particular, the transducer array may have a chip design. The transducer array elements are units, which may be used as an actuator for generating an ultrasound pulse or signal. Generally speaking, one transducer array element may consist of one or more transducer sub-elements. A single transducer can be a piezoelectric micro-machined ultrasonic transducer (pMUT) component or a capacitive micro-machined ultrasonic transducer (cMUT) component.

In order to reduce the complexity of the system, several transducer array elements may be combined into a, e.g., one, functional unit and treated as a single "element", i.e., the combined transducer array elements are driven at that same time as if they are a single unit.

The transducer array elements in the transducer array may be connected in series or parallel, such that they can be driven optimally.

The transducer array may be flexible. Thus, the system can advantageously adjust its geometry to a probe or sample surface, e.g., a patient's body. Thus, the array can assume different shapes, wherein the position and/or orientation and/or pose of an individual transducer array element relative to another transducer array element and/or relative to the transducer array as a whole is deviating from a predetermined or expected position or pose.

The transducer array may be integrated into an adhesive patch for fixing the transducer array to a patient's body. The system can then be advantageously and easily used, e.g., for monitoring a patient's body functions, such as cardiac functions, especially for long-term monitoring. The transducer array may be integrated into the patch by providing it with an adhesive area, which is suited for attaching the patch to a patient's body surface. Also, a textile or other flexible material may be provided with an adhesive material or simply a mechanical strap without adhesive or a combination thereof and used to fix the transducer array to the body surface. In particular, the patch may be configured such that it secures the transducer array in close proximity to the body surface.

The plurality of sub-arrays is defined as a collection of one or more transducer array elements and are controlled as functional units for sending ultrasound pulses and/or receiving ultrasound signals. In this respect, rather than operating the transducer array as a single whole and using each element as an individual unit, a sub-array scanning architecture may be used, i.e., groups of transducer array elements may be combined into functional units. In particular, neighboring elements may be combined. A pulse-echo measurement performed by an active sub-array in one single angle/direction may be referred to as a shot. Hence, a scan sequence may be a collection of shots, with or without an activation order. In other words, a scan sequence may be a collection of active sub-arrays (e.g. for performing at least one pulse-echo measurement) or shots, with or without an activation order.

The system may also comprise at least two transducer arrays forming a least one transducer array set; and the control unit may be configured to control the transducer array set as one single transducer array. By using a transducer array set for example several positions can be monitored (e.g. needed for lung monitoring) by means of one system. Further, a greater surface area can be monitored. Also, different viewpoints for monitoring a target, which can be an organ, fluid carrying structure or other volumetric feature in the body e.g. the heart and/or lung and/or chest, can be established. The transducer array units may be combined in series and/or in parallel into a single element, i.e., a single transducer array set, which is driven as a single transducer array of the ultrasound system.

The control unit may comprise several components, which may be structurally integrated with each other, for example in a common casing or envelope structure, or which may be implemented as structurally separated components, which may be coupled to each other.

The transducer array elements are configured to generate and/or receive ultrasound, in particular ultrasound pulses or signals, based on a control signal. To provide the respective control signals for the transducer array, the control unit may comprise a send module and a receive module, which may be implemented in a driver module for the transducer array and/or for transistors, which may control the transducer array elements.

The control unit may be configured to perform different tasks such as, but not limited to, the following examples: The control unit may be configured to perform digital signal processing and/or filtering. Also, the control unit may be configured to perform operations related to storing and/or retrieving information, in particular in a memory module, such as a random-access memory (RAM) module. Also, the control unit may be configured to perform operations for driving an external interface, such as an interface to a memory module, a transceiver unit, e.g., via a USB interface or a wireless interface, and/or receiving control signals from other units. The control unit may also perform operations for controlling the transducer array, e.g., through a switch linked to a transducer array element, such as a switch can be a transistor or more specifically a thin-film-transistor (TFT) element, or a matrix of such switches/transistors, e.g., in a TFT matrix. The TFT matrix can be a(n) (integral) part in terms of function and/or structure of the control unit. Also, the control unit may be configured to control a TFT element connected to a transducer element, to generate delays for generating and/or sensing ultrasound pulses and signals, to define generated ultrasound pulses, beam-forming operations, and/or to control power management. Each of said functions may be carried out and/or implemented in an ASIC design individually or in combination. Also, these functions may be implemented as modules of the control unit.

The control unit may comprise a switch, e.g., a transistor or thin-film transistor (TFT), which is configured to control a transducer array element. In particular, an array of switches, e.g., thin-film transistors may be provided, which is coupled to the transducer array. In particular, the TFT switch array (e.g., TFT array) may be structurally integrated with the transducer array in one component, e.g., in a common casing or envelope structure.

The control unit may consist of or comprise one or several microprocessors. Also, the control unit may comprise an application-specific integrated circuit (ASIC) design. The control unit, in particular a microprocessor of the control unit, may be fully or partially integrated into a printed circuit board (PCB).

The system may comprise a field programmable gate array (FPGA) and/or a digital application-specific integrated circuit (ASIC), configured such that one or more control algorithms for the transducer array are carried out. The FPGA and/or ASIC may be included in the control unit. In other words, the control unit may comprise the FPGA and/or ASIC.

The data processing unit may be implemented in different ways. It may for example comprise a microprocessor. In particular, it may comprise an ASIC design. Also, the data processing unit and the control unit may be implemented as software modules that are run, at least partially, on a common computational unit. For example, a processing unit may read from a memory and execute instructions for performing operations to implement the data processing unit and/or control unit. Also, the data processing unit may comprise one or several analog-digital converters (ADC) and/or digital-analog converters (DAC). These converters are configured to link analog and digital channels to each other and allow both digital data collection and treatment, and treating analog control and detection signals. A time gain controller may be implemented using a DAC element and/or a different architecture.

The feature detection module may be implemented as a software module, comprising instructions for computational operations that implement the function of feature detection, in particular the determination step. This software module may in particular be implemented as a module of the control unit. Specifically, the feature detection module may be comprised, at least partially, by the control unit and/or by the data processing unit. Additionally or alternatively, the feature detection module may be implemented on a computer, e.g., a PC, a mobile device, a network and/or a cloud system, each being separately configured with respect to the transducer array, the control unit and/or the processing unit, wherein, in this case, the ultrasound system may further comprise an interface unit configured for interfacing, e.g., interfacing via cable and/or wirelessly, with the feature detection module, e.g., for exchanging data (e.g., transducer array data and/or detection information (data)).

The coupling between the transducer array and the control unit may comprise a wire connection, in particular an interface for data and/or power transmission between the transducer array and the control unit. In particular, the control unit and/or a part of the control unit may be arranged on the sensor array or may be integrated with the sensor array into one common part. The sensor array may be embodied especially by means of the combination of a switch (e.g. TFT) array and a transducer array. In particular, there may be a wire connection and/or a wireless interface provided, if the control unit controls the sensor array. If a wireless sensor array is implemented, the control unit may be arranged on the sensor array and/or integrated into one component with the sensor array. In particular, if the sensor array is connected to another element by a wire, at least part of the controlling of the sensor array may be implemented on the sensor array and/or integrated into one component with the sensor array. Also, an additional control unit may be provided, e.g., in a separate PC or control unit, when there is access through wires to the sensor array. A sensor array is for example a combination of a transducer array and a TFT array. Parts of the controller can be integrated on a TFT array by an integrated circuit design or by bonding ICs to the TFT.

The ultrasound system may further comprise a power source unit for providing energy to the transducer array and/or the control unit. For example, the power source may comprise a battery, which is integrated in a portable device as a component of the system.

The scan sequence module may be a combination of hardware control logic for selecting sub-array positions/shots and software modules that implement the scan sequence step. The scan sequence module may be implemented, at least partially, in and/or as a part of the control unit and/or the data processing unit. To assure a good field of view of the region of interest for the scan sequence module, a procedure may be included for patch placement (e.g., prior to operating the scan sequence module and/or to performing the scan sequence step) that can help to maximize the chances of having a good field of view on the region of interest, i.e. a clear external body reference for placing the patch for cardiac monitoring may be provided as part of the procedure.

Alternatively, or additionally, the scan sequence module may be implemented as a software module, comprising instructions for computational operations that implement at least the function of the scan sequence step. This software module may in particular be implemented as a module of the control unit. Specifically, the scan sequence module may be implemented, at least partially, by the control unit and/or by the data processing unit. In other words, the control unit and/or the data processing unit may, in particular at least partially, comprise the scan sequence module. Further, additionally or alternatively, the scan sequence module may be implemented, at least partially, on a computer, e.g., a PC, a mobile device, a network and/or a cloud system, each being separately configured with respect to the transducer array, the control unit and/or the processing unit, wherein, in this case, the ultrasound system may further comprise an interface unit configured for interfacing, e.g., interfacing via cable and/or wirelessly, with the scan sequence module, e.g., for exchanging corresponding data (e.g., data for performing the scan sequence step and/or for operating the transducer array on the basis of the scan sequence step).

The scan sequence may be a sequence of sub-array positions, at which pulse-echo measurements (also called shots) are performed. The scan sequence may be meant to scan/measure/image the region of interest and, accordingly, the feature of interest. In general terms the scan sequence may refer to any activation sequence of transducer elements, during send and receive, with the purpose of gathering the measurement data, i.e., transducer array data, that may be used for establishing a 3D/2D or 1D image of the region of interest and, accordingly, of the feature of interest.

Generally, a typical scan sequence could be a regular grid of sub-array positions, where for each sub-array position a number of measurements may be performed for different "beam-directions". This may be done by measuring all possible positions or by performing a sparser stepping of the possible positions. This would however lead to suboptimal usage of the available resource as many of these measurements would contain areas, which are not necessarily related to the area of interest. Therefore, smarter scan sequences, i.e., a smarter scan sequence step, may be integrated in the scan sequence module, which may lead to a more efficient scanning of the region of interest and, accordingly, the feature of interest, or may provide a good distribution of measurements over the region of interest and, accordingly, the feature of interest with minimal amount of shots.

Hence, additionally or alternatively, the scan sequence step may comprise at least one initial scout scanning for gathering ultrasound data for detecting at least one region of interest, and/or at least one subsequent main scanning for only gathering ultrasound data of the region of interest. In other words, due to performing an initial "rough" scouting scan, general regions of interest may be detected, which may then be scanned in a more detailed manner, i.e., the scouting scan may be followed by a more detailed scan of the region of interest. In particular, the detailed scan may be personalized towards the specific body topography of subject, i.e., the individual patient.

Additionally, or alternatively, the scan sequence step may comprise at least one initial scanning for gathering ultrasound data for detecting at least one region of interest based on general stochastic or quasi-stochastic information about a location of the region of interest, and at least one subsequent main scanning for gathering ultrasound data of the region of interest. In other words, the shot locations may be selected based on a stochastic or quasi-stochastic process. Due to the stochastic or quasi-stochastic nature of the process, an initial "noisy" or "grainy" image may be produced, which may however already be used to detect general regions of interest. Then a more detailed scan of the region of interest may be performed. Furthermore, for example, in an iterative process said initial "noisy" or "grainy" image may be refined and the analysis repeated every "N" step, until a threshold is reached in a feature detection module (which is described in more detail further below), e.g., based on convergence, number of iterations and/or another pre-defined metric.

Additionally, or alternatively, the initial scout scanning and/or the main scanning may be based on general stochastic or quasi-stochastic information about a location of the region of interest.

Additionally, or alternatively, the scan sequence step may comprise at least one scanning for gathering ultrasound data of at least one region of interest based on an activation of a sequence of sub-arrays according to a rank-order of the sub-arrays, in particular of the sub-array positions including shot direction. In other words, a rank-order of all the possible sub-arrays/shots may be generated and the sub-arrays/shots may be chosen according to this rank order for gathering ultrasound data. This choice of the sub-arrays/shots (i.e., according to the rank-order) may characterize all characteristics of the feature (e.g., tissue) of interest in the best possible way.

The rank-order of the sub-arrays may have a rank-order limitation. In particular, a total budget of S sub-arrays/shots may be given, wherein the scan sequence module may choose which are the best S sub-arrays/shots (out of a much large number of possible sub-arrays/shots, e.g., all sub-arrays). Hence, the scan sequence module may choose a scan sequence for gathering ultrasound data. This choice of the best S sub-arrays/shots may characterize all features of the tissue of interest in the best possible way.

The scan sequence step may comprise determining the rank-order of the sub-arrays based on a coverage information and a regularity information achievable by the sub-arrays, when operated. In particular, as to the regularity of the shots, regularly spaced shots may tend to produce better images that are easier to interpolate. In particular, the coverage may be defined as the ratio of volume of the tissue of interest that can be observed to the total volume of the feature (e.g., the tissue) of interest.

The regularity may be defined as the average distance between all points in the tissue of interest, i.e., the feature of interest, and a specific shot.

Additionally, or alternatively, the coverage may be defined as the ratio of length/area/volume of the tissue of interest, i.e., the feature of interest, that can be observed to the total length/area/volume of the feature (e.g., the tissue) of interest.

In particular, when applied to area or volume the coverage, additionally or alternatively, may be defined as the area/volume of the convex hull formed by the intersection of shots origination from all sub-arrays in the scan sequence and the tissue of interest, i.e., the feature of interest. The convex hull may be a collection of points in which the smallest convex set includes all points of the feature of interest FOI.

The coverage information and the regularity information may be obtainable by at least one initial scout scanning for gathering ultrasound data and/or by predetermined topography and/or location information.

Optionally, the initial scout scanning and/or the main, e.g., detailed, scanning, in other words: the scanning according to the rank order, may additionally be done stochastically or quasi-stochastically. In other words, said scanning may particularly be based on stochastic or quasi-stochastic information about a location of the region of interest.

The system may further comprise at least one reference module configured for obtaining reference ultrasound data, wherein the reference ultrasound data is obtained from the ultrasound data gathered by the scan sequence module.

The system may further comprise at least one feature detection module configured for performing a determination step of the ultrasound data gathered by the scan sequence module to detect at least one feature of interest. The detection information may comprise, e.g. all, information on the detected features, such as position, orientation, size and/or shape.

The feature detection module may be configured to interface with the reference module and to provide at least one detection information to the reference module determined by the determination step.

The feature detection module may be configured to, based on the detection information, establish and provide the reference ultrasound data to the reference module and/or provide at least one region of interest of the at least one feature of interest to the reference module, to thereby allow the reference module to subsequently establish the reference ultrasound data (e.g., on the basis of a scan sequence of the scan sequence module, e.g., a rank-order of sub-arrays). Alternatively, or additionally, at least one region of interest of the at least one feature of interest may be supplied directly to the scan sequence module, which may execute a, e.g., the, scan sequence and may provide the measured data to the reference module to subsequently establish the reference ultrasound data.

One of the functions of the feature detection module may particularly be that, by performing an analysis of features inside the sensor's field of view, i.e., a determination step of the transducer array data, with a/the feature detection module, detection information may be generated. The detection information may be used by the scan sequence module to provide further basis to optimize the generation of scan sequences, alternatively or additionally the detection information data might also be transmitted to the reference module, to thereby provide the reference module with a basis, on which the reference module may further process the transducer array data in a selective and well-directed manner in dependence of the detected features of interest. Hence, due to the detection information obtained by the feature detection module, features of interest, in particular organ features, may be identified and discerned as well as it may be evaluated if the targeted features of interest are visible in the sensor's field of view. Accordingly, feedback (e.g., user feedback) regarding said features of interest themselves as well as regarding the correctness of the transducer array placement may be provided. In other words, a feature detection module may be provided which may perform a determination step of the transducer array data, which represent features inside the transducer array's field of view, wherein the determination step may help to identify organ features, but also evaluate if the targeted organ features are visible in the field of view, thus being able to provide critical (user) feedback on the correctness of the patch placement.

In this feature detection module, the detection of the feature of interest, e.g., the left ventricle (LV) within a cardiac scan, may be a key step, which may serve not only to optimize and personalize the scanning process of the ultrasound system, but may also enable automated analysis of the feature's size and function.

Further, the feature detection module may facilitate a more focused ultrasound monitoring on a feature of interest instead of constant monitoring of an unrestricted area by the whole transducer array, e.g., in an automated manner. Accordingly, a power saving mode may be, e.g., automatically, enabled. The use of the feature detection module, i.e., the use of the detection information gained by the feature detection module, may enable to restrict monitoring on the region of interest of the feature of interest such as an organ, fluid carrying structure or other volumetric feature in the body, e.g. the heart, or a part of a target organ, in particular a ventricle or atrium, which during a cardiac cycle or selected phases of a cardiac cycle provides the required physiological and/or pathological information. For instance, the volume of the left ventricle instead of the volume of the total heart may be monitored to determine how much volume of blood the heart is pumping, in particular per cardiac cycle or a selected phase of a cardiac cycle. Overall, through this (e.g., automatic) restriction of monitoring, a less power consuming monitoring and a longer lifetime of the battery of the system and/or less frequent charging may be enabled. In other words, in addition to the items pointed out further above, the system may advantageously require less computational capacities and the power consumption of the ultrasound system may be reduced.

The scan sequence module may be configured to interface with the feature detection module and/or the reference module for providing the ultrasound data to the feature detection module and/or the reference module (e.g., for detecting the feature of interest or for obtaining the reference ultrasound data).

Alternatively or additionally, the scan sequence module may be configured to interface with the feature detection module and/or the reference module for providing measurement data based on the executed scan sequence to the feature detection module and/or the reference module. Thereby, the feature detection module may be allowed to perform a determination step for detecting at least one feature of interest and/or to thereby allow the reference module to obtain the reference ultrasound data respectively based on the ultrasound data obtainable by scanning on the basis of said scan sequence. In other words, the scan sequence module may provide a/the scanning sequence, which may be used to gather ultrasound data useable as input for either the reference module and/or the feature detection module.

In particular, the feature detection module may be implemented as a software module, comprising instructions for computational operations that implement the function of feature detection, in particular the determination step. This software module may in particular be implemented as a module of the control unit. Specifically, the feature detection module may be implemented, at least partially, by the control unit and/or by the data processing unit. In other words, the control unit and/or the data processing unit may, in particular at least partially, comprise the feature detection module. Additionally or alternatively, the feature detection module may be implemented, at least partially, on a computer, e.g., a PC, a mobile device, a network and/or a cloud system, each being separately configured with respect to the transducer array, the control unit and/or the processing unit, wherein, in this case, the ultrasound system may further comprise an interface unit configured for interfacing, e.g., interfacing via cable and/or wirelessly, with the feature detection module, e.g., for exchanging data (e.g., transducer array data and/or detection information (data) and/or scan sequence data).

At least one sub-array of transducer array elements may be defined and controlled as a functional unit for sending ultrasound pulses and/or receiving ultrasound signals. Rather than operating the transducer array as a single whole and using each element as an individual unit, a sub-array scanning architecture may be used, i.e., groups of transducer array elements may be combined to functional units. In particular, neighboring elements may be combined. The system may comprise at least two transducer arrays forming a least one transducer array set; and the control unit may be configured to control the transducer array set as one single transducer array. By using a transducer array set for example several positions can be monitored (e.g. needed for lung monitoring) by means of one system. Further, a greater surface area can be monitored. Also, different viewpoints for monitoring a target, which can be an organ, fluid carrying structure or other volumetric feature in the body e.g. the heart and/or lung and/or chest, can be established. The transducer array units may be combined in series and/or in parallel into a single element, i.e., a single transducer array set, which is driven as a single transducer array of the ultrasound system.

The feature detection module may function by analyzing the characteristics of the received ultrasound signal or the image data derived from these ultrasound signals, in particular the transducer array data, and deriving so-called (feature) detection information based on the analysis of these signals / the transducer array data. The detection information may be the information needed for the reference module to obtain reference ultrasound data. The process of generating the detection information is also called the determination step.

The feature detection module may be configured to, based on the detection information, establish and provide the reference ultrasound data to the reference module and/or provide at least one region of interest of the at least one feature of interest to the reference module, to thereby allow the reference module to subsequently establish the reference ultrasound data.

In other words, based on the detection information, reference ultrasound data may be received, obtained, calculated and/or computed by the reference module.

In particular, when the transducer array is placed on the body (e.g. the thorax) of the subject, it does not have any prior information on the features of the body on which it is placed. Thus, after scanning (e.g., on the basis of a scan sequence of the scan sequence module, e.g., a rank-order of sub-arrays), the feature detection module may analyze the characteristics of the received ultrasound signal or the image data derived from these ultrasound signals, in particular the transducer array data, for detecting the features of interest and generate the detection information. Subsequently, when the results satisfy a predetermined requirement (e.g., a high resolution, in particular a resolution being sufficiently high for further processing), the feature detection module may establish and provide the reference ultrasound data to the reference module. Alternatively, or additionally, when the results do not satisfy a/the predetermined requirement (e.g., a high resolution, in particular a resolution being sufficiently high for further processing), the feature detection module may provide at least one region of interest of the at least one feature of interest to the reference module and/or scan sequence module. The reference module may then establish reference data based on a detailed scanning of said region (e.g., on the basis of a scan sequence of the scan sequence module, e.g., a rank-order of sub-arrays). Hence, this scanning will allow to detect the feature of interest / the target (e.g., target organ), and, accordingly, reference ultrasound data may be obtained.

The feature detection module may further be configured to, in the determination step, determine whether the feature of interest is located in the field of view of the transducer array. This way, a plausibility check of the data can be performed. Further, feedback information may be generated to be the basis for a critical user feedback, to thereby allow a/the user to correct the positional placement of the transducer array for gaining better and/or more complete ultrasound imaging results of the targeted features, i.e., the features of interest. Inter alia, this embodiment of the feature detection module allows to perform an analysis of features inside the sensors field of view. This analysis can help to identify organ features, but also to evaluate (automatically), if the needed organ feature is visible in the field of view, thus being able to provide critical user feedback on the correctness of the patch placement.

The system may further comprise a feedback module configured for providing a request for repositioning the transducer array based on the determination step.

The feedback module may be implemented as a software module, comprising instructions for computational operations that implement the function of feedback, e.g., the provision of a request for repositioning the transducer array. This software module may in particular be implemented as a module of the control unit. Specifically, the feedback module may be comprised, at least partially, by the control unit and/or by the data processing unit. Additionally or alternatively, the feedback module may be implemented, at least partially, on a computer, e.g., a PC, a mobile device, a network and/or a cloud system, each being separately configured with respect to the transducer array, the control unit and/or the processing unit, wherein, in this case, the ultrasound system may further comprise an interface unit configured for interfacing, e.g., interfacing via cable and/or wirelessly, with the feedback module, e.g., for exchanging data in order to be able to provide a request for repositioning the transducer array.

Due to the feedback module, a/the user may be requested to reposition the transducer array on the body of the subject to improve the visibility of the feature of interest, in order to alter the geometrical relation between the transducer array and the position of the feature of interest.

The determination step may comprise
- determining and extracting relevant feature points, in particular relevant feature points with respect to the feature of interest, from the transducer array data by phase-based and/or morphological image processing, wherein the transducer array data are receivable by the feature detection module, when an ultrasound pulse is generated by at least one transducer array element and an ultrasound signal is subsequently received by the same and/or another transducer array element (e.g., generated and received by sub-arrays in accordance to a/the scan sequence, e.g., a/the rank-order),
- aligning the relevant feature points with at least one predetermined information representing a geometrical model of the feature of interest, and
- evaluating the match between the relevant feature points and the at least one predetermined information, to thereby generate the detection information comprising the feature of interest and its associated region of interest.

The at least one predetermined information may comprise at least one simple geometrical shape, such as an ellipsoid, and/or at least one template model derived from one or more predetermined features of interest of one or more subjects and/or at least one template model being based on statistical shape analysis of a predetermined population (i.e., larger group of subjects being connected by at least one characteristic, e.g., age, gender, etc.).

By means of the evaluation step of the match between the relevant feature points and the at least one predetermined information, to thereby generate the detection information comprising the feature of interest and its associated region of interest, the handling of the overall system becomes more secure and reliable even for untrained users and to ensure a correct placement of the transducer array of the ultrasound system.

The at least one predetermined information may comprise myocardial tissue information and/or myocardial tissue interface information, in particular the at least one predetermined information may comprise left ventricle information. This is specifically of advantage, when monitoring the heart and more specifically the left ventricle.

The determination step may further comprise, before aligning, skeletonizing the relevant feature points for reducing the number of feature points. Hence, calculations may be speeded up and, accordingly, processing time may be brought to a time frame, which, e.g., may be compatible with online use on monitoring applications such as the present ultrasound system, since, instead of aligning all the feature points, i.e., the full point cloud, of the detected features, pre-processing may be performed using a skeletonization operator, i.e., skeletonizing may be performed, that reduces the number of feature points in the target feature shape cloud while keeping good alignment accuracy.

Additionally, evaluating the match of the determination step may further comprise evaluating whether the feature of interest is completely represented by the relevant feature points, to thereby determine whether the feature of interest is located in the region of interest. This way, the plausibility check and the verification of correct placement of inter alia the transducer array of the ultrasound system can be further enhanced. If the relevant feature points can be verified, this is a solid basis for a verification and helps to confirm the correctness of the setup.

Additionally, evaluating the match of the determination step may further comprise determining a region of the region of interest, where relevant feature points are missing, based on the at least one predetermined information.

Optionally, evaluating may further comprise detecting parts in the transducer array data being occluded by the presence of different features, such as rib/bone/lungs. Hereto, the existence of sufficient image contrast may be estimated by taking different metrics, such as the intensity of the image gradient and/or the value of the dynamic range of the transducer array data, in particular possibly not in the entire image to improve performance (e.g., discarding near field data or calculating only at depths beyond 6 cm). Automatic discarding of the occluded regions may be done by setting an automatic threshold, which may be estimated via one or more of different existing methods, e.g., in particular, Otsu's method. To estimate this, values of the selected metrics, such as dynamic range values of the images based on the transducer array data taken over the entire extent of the transducer array, may be used to feed the Otsu's method to ensure that both non-occluded and occluded regions are considered for the estimation of the threshold value.

The system may further comprise at least one volume reconstruction module configured for reconstructing the volume of a target organ or part of a target organ.

The target organ may be the feature of interest, and vice versa.

The target organ may be the heart, left/right ventricles, left/right atria, blood vessels, aorta, lungs, etc.

The volume reconstruction module may be a software module and may in particular be implemented together with the control unit. Specifically, the volume reconstruction module may be comprised, at least partially, by the control unit and/or by the data processing unit.

The volume reconstruction module may reconstruct the volume of the target organ / feature of interest, e.g., the left ventricle, based on the reference ultrasound data obtained by the reference module.

The system may further comprise at least one selected monitoring unit configured for monitoring the volume of the target organ and/or part of the target organ.

Central to the selected monitoring unit may be the fact that not the entire volume of the feature (i.e., target organ or part of a target organ) is traced, and not the entire transducer array is used (thus not all transducer elements are used for tracing). In this respect, the selected monitoring unit may be configured for selective monitoring based on the feature of interest detected by the determination step.

Referring to the issues as pointed out further above, the invention also provides a method for ultrasound imaging of a body part of a subject according to claim 12 of the attached set of claims for solving the discussed problem. Further advantageous embodiments are given in the dependent claims.

The method for ultrasound imaging of a body part of a subject comprising at least the following steps:
- placing a transducer array with a plurality of transducer array elements for providing ultrasound waves for transmission into the body of the subject, and
- performing a scan sequence step for obtaining ultrasound data based on reception of the ultrasound waves transmitted into the body of the subject.

Additionally, or alternatively, the method of ultrasound imaging of a body part of a subject may comprise the following steps:
- obtaining transducer array data based on reception of the ultrasound waves transmitted into the body of the subject,
- performing a determination step of the transducer array data to detect at least one feature of interest, and
- obtaining reference ultrasound data based on at least one detection information determined by the determination step.

In particular, the method may serve the purpose of operating the ultrasound system. Thus, it may have the same features and/or the same advantages as the ultrasound system of the invention.

The scan sequence step may comprise at least one scanning for gathering ultrasound data of at least one region of interest based on an activation of a sequence of sub-arrays according to a rank-order of the sub-arrays, wherein, optionally, the rank-order of the sub-arrays has a rank-order limitation (as already outlined above).

The placing of the transducer array may further comprise aligning the transducer array based on at least one landmark of the body of the subject, wherein, optionally, the landmark is a rib and/or the sternum of the subject.

As described above, the method may further comprise performing a determination step of the obtained ultrasound data to detect at least one feature of interest, and obtaining reference ultrasound data based on at least one detection information determined by the determination step.

The method may further comprise providing a request for repositioning the transducer array based on the determination step. This helps especially untrained, but also well-trained users of the system, as this makes the handling of the system very simple and intuitive. No complex verification or the like is needed, the system itself will inform the user, whether a repositioning of the transducer array is needed or not.

The method may further comprise reconstructing the volume of the target tissue or anatomical structure and/or part of the target organ. This way, the monitoring and/or imaging by the ultrasound system can be further enhanced. Also, data provided by the ultrasound system are than easier to interpret, thereby allowing also untrained users a secure and easier handling of the system.

The method may further comprise monitoring the volume of the target tissue or anatomical structure and/or part of the target tissue or anatomical structure. With this capability, the system allows an easier detection of abnormal or pathophysiological changes to the organ and/or the target tissue.

The invention will be better understood with the aid of the description of an embodiment given by way of example and illustrated by the figures.

It is shown in
- **Fig. 1**: an embodiment of the ultrasound system;
- **Fig. 2A-B**: detailed views of the transducer array of the embodiment of the ultrasound system;
- **Fig. 3**: another embodiment of the ultrasound system;
- **Fig. 4**: another embodiment of the ultrasound system;
- **Fig. 5**: an embodiment of a method to operate the ultrasound system;
- **Fig. 6**: another embodiment of a method to operate the ultrasound system;
- **Fig. 7**: a visualization of an exemplary image obtained on the basis of the method of Fig. 5;
- **Fig. 8**: an example of an ultrasound simulation obtained from the image shown in Fig. 7;
- **Fig. 9**: a visualization of an example of a sub-array selection based on the method of Fig. 5;
- **Fig. 10**: a visualization of a skeletonizing step of a determination step of another embodiment of a method to operate the ultrasound system; and
- **Fig. 11A-B**: a visualization of a feature of interest detected on the basis of the disclosure with respect to Figs. 4, 5 and/or 6.

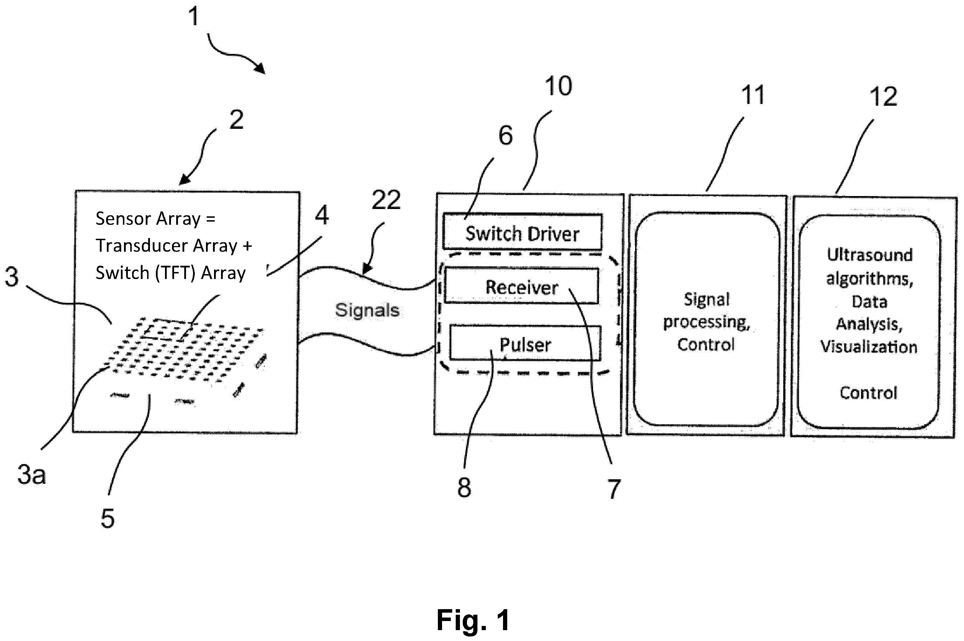

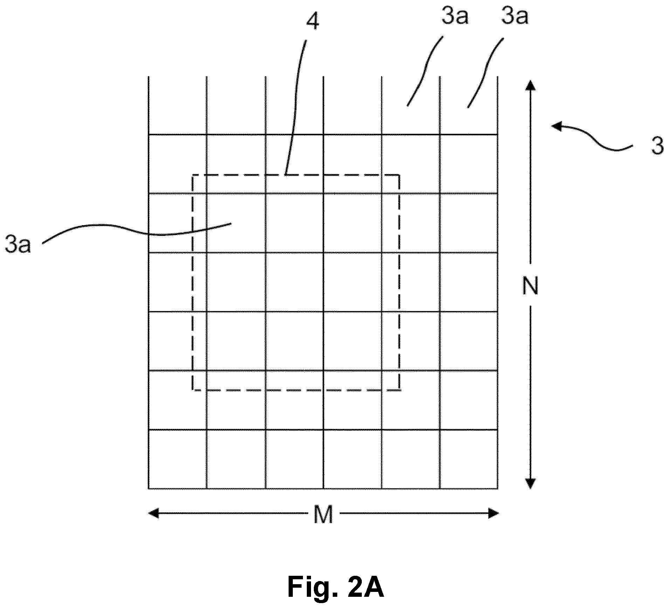

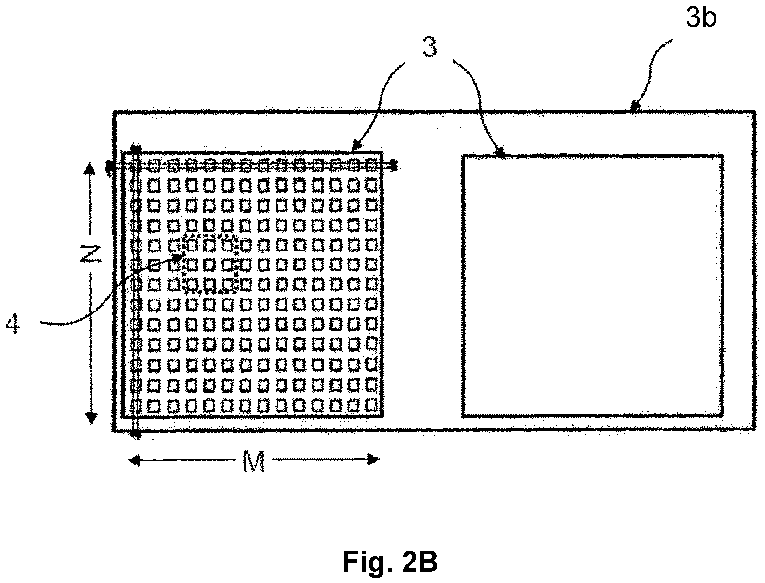

Turning to **Fig. 1****,** **Fig. 2A** and **Fig. 2B****,** an embodiment of the ultrasound system is described.

**Fig. 1** shows a schematical overview and explaining the functional blocks of the ultrasound system 1.

The ultrasound system 1 according to the embodiment is configured as a wearable ultrasound system 1, in particular for long-term monitoring of a patient's cardiac activity.

The ultrasound system 1 comprises a sensor array 2 and support electronics that can be integrated in a flexible body worn format, e.g., in a patch.

In the embodiment as shown in **Fig. 1****,** the ultrasound system 1 can be (at least partially) carried in a patch 52 (not shown).

The sensor array 2 comprises a transducer array 3 including a plurality of transducer array elements 3a. The plurality of transducer array elements 3a are arranged in a 2D matrix having N rows and M columns as shown in Figs. 2A and 2B. The number of the rows and columns may be equal or different.

For example, the transducer array elements 3a may comprise a plurality of subelements, wherein each subelement is an individual piezoelectric micromachined ultrasonic transducer (pMUT).

In other embodiments, i.e., alternatively, the transducer array 3 may comprise a plurality of capacitive micro-machined ultrasonic transducers (cMUT).

In particular, it may be conceivable that each transducer array element 3a may consist of one or several sub-elements, in particular pMUT sub-elements or components, which are driven (through corresponding electronics or electrical interconnects) as a single larger transducer array "element" 3a. For example, an array element 3a may consist of one or several pMUT sub-elements.

A subset of the transducer array elements 3a forms a sub-array 4. In the present case, a plurality of sub-arrays 4 are formed, wherein only one sub-array 4 is shown in Figures 1, 2A and 2B for reasons of clarity. A sub-array 4 is defined as an Ns x Ms array of transducer array elements 3a that may act together for purposes of beamforming and image formation, wherein Ns may be equal to or unequal to Ms. In the present case, N is equal to M and Ns is equal to Ms, wherein N >> Ns.

Furthermore, the sensor array 2 comprises a switch array (or a switch module) 5. The switch array 5, for example, can be embodied as a TFT array.

As illustrated in Fig. 1, the ultrasound system 1 further comprises one or more, e.g., a plurality, of units, e.g., functional units and/or modules and/or blocks, for processing and controlling signals 22 received from the sensor array 2. The units are schematically illustrated in Fig. 1, e.g., as functional blocks, and include a front-end block 10, a digital middle-end block 11 and a digital back-end block 12.

The front-end block 10 comprises a switch driver module 6 for driving the switch array 5 of the ultrasound sensor array 2, a receiver module 7 for converting an analog input signal to a digital output signal, and a pulser module 8 for generating ultrasound pulses.

For example, the switch driver module 6 can be embodied as a TFT driver module, and the receiver module 7 can be embodied as an analog-digital converter (ADC).

Additionally, the ultrasound system 1 may further comprise an embedded compute system digital application-specific integrated circuit (ASIC) for supporting the sensor array 2 and an interface unit (i.e., e.g., an interface module) which is configured to allow for data communication and/or power connections with external units (not shown).

In particular, the sensor array 2 (including the transducer array 3) and the switch/TFT array 5, is coupled with the switch driver module 6, the receiver module 7 and the pulser module 8 as shown in Fig. 1.

These modules 6, 7, 8 are configured to communicate via signals 22 with the sensor array 2. The signals 22 may be digital or analog signals.

In embodiments, the modules 6, 7, 8 may be implemented in application-specific integrated circuits. Such ASICs can be located on the periphery of the sensor array 2 or on a PCB, in particular a flexible PCB.

The modules 6, 7, 8are configured to receive and to treat, i.e., process, the signals 22 provided by the sensor array 2.

The digital middle-end block 11 of the ultrasound system 1 includes a signal processing/control that is in communication with the switch driver module 6, the receiver module 7 and the pulser module 8.

The digital middle-end block 11, i.e. the signal processing/control unit thereof, which can be, for example, implemented in an embedded compute system which is integrated in the ultrasound system 1.

The embedded compute system may be implemented on with a (e.g., flexible) PCB (e.g., a CPU, or a FPGA including memory and/or processing units). The embedded compute system may also be implemented partly or completely in an ASIC.

The processed signals received from the signal processing unit of the digital middle-end block 11 are further processed by the processing algorithms provided in the digital back-end block 12 of the ultrasound system 1. The processing algorithms for example include ultrasound image reconstruction algorithms, data analysis algorithms, visualization algorithms and/or control algorithms.

The digital back-end block 12 may be implemented in a wired external unit or in a wireless unit. In the case the digital back-end block 12 is implemented in an externally wired unit such as a PC, at least part or all of the treatment algorithms can still be run on the embedded compute system. In the case the digital back-end block 12 is implemented as a wireless unit, the processing algorithms can be incorporated in the embedded compute system integrated in the ultrasound system 1. Alternatively, it is also envisaged that at least a part of the processing algorithms may be incorporated in a wearable compute system, e.g. similar to a holter monitor for ECG.

The ultrasound system 1 further comprises a scan sequence module 42 (not shown in Fig. 1) configured for performing a scan sequence step, wherein the scan sequence module 42 is configured to activate a sequence of sub-arrays 4, during the scan sequence step, for gathering ultrasound data.

The ultrasound system 1 further comprises a reference module 43 (not shown in Fig. 1) configured for obtaining reference ultrasound data, the reference ultrasound data being obtained from the ultrasound data gathered by the scan sequence module 42, and a feature detection module 44 (not shown in Fig. 1) configured for interfacing with the reference module 43 and for performing a determination step of the ultrasound data gathered by the scan sequence module 42, e.g., the transducer array data, to detect at least one feature of interest FOI.

The scan sequence module 42 is implemented as a combination of the above-described hardware control logic for selecting sub-array positions and a software module that implement the scan sequences.

Both, the reference module 43 and the feature detection module 44, are implemented as software modules, each comprising instructions for computational operations that implement their intended function.

In the present embodiment, these software modules are implemented as modules of the back-end block 12. It might also be possible that said modules may be a respective part of the back-end block 12.

A more detailed description regarding the scan sequence module 42 as well as regarding the reference module 43 and the feature detection module 44, configured to provide at least one detection information to the reference module 43, will be given further below.

Turning to **Fig. 2A****,** a detailed view of the transducer array 3 of the embodiment of the ultrasound system 1 is described. The transducer array 3 has a matrix of transducer elements 3a, which are only schematically shown in a small number.

In particular, it may be conceivable that each transducer array element 3a may consist of one or several sub-elements, in particular pMUT sub-elements or components, which are driven (through corresponding electronics or electrical interconnects) as a single larger transducer array "element" 3a. For example, an array element 3a may consist of one or several pMUT sub-elements.

The system 1 is configured such that a plurality of sub-arrays 4 of transducer elements 3a are defined (only one is shown for reasons of clarity). The elements 3a of the sub-array(s) 4 are treated as one functional unit for generating ultrasound pulses and/or detecting ultrasound signals.

Furthermore, **Fig. 2B** shows another embodiment, wherein two or more transducer arrays 3, e.g., a first and a second transducer array 3, can form a transducer array set 3b, which is then controlled as one functional unit, i.e., "virtual" single transducer array 3.

**Fig. 2B** also shows that elements 3a along a row and/or a column of the transducer array 3 are interconnected with one or more interconnecting lines, a so-called row/column-based architecture. The elements 3a connect in parallel to the interconnects, with a switch, in particular a TFT switch, determining if an element 3a is connected or not and to which signal path it connects. Thus, rows and/or columns of the transducer array 3 can be controlled together. This allows simplifying the system by switching rows and/or columns of elements 3a together.

Furthermore, the ultrasound system 1 comprises a switch array which can be embodied as a thin-film transistor (TFT) array 5, comprising a plurality of TFT elements.

The switch array 5 is arranged such that its switch elements (or TFT elements when embodied as a TFT array) control corresponding transducer elements 3a. In particular, each TFT element is assigned to one transducer element 3a. The array size depends in particular on the size of the respective pMUT array 3, and it may differ in different embodiments.

In the embodiment, both the transducer array 3 and the TFT array 5 may be integrated into an adhesive patch 52, in particular together with other wearable parts of the system In the embodiment, the adhesive patch 52 may have a size of about 10x10 cm. The size may vary in different embodiments.

The adhesive patch 52 may comprise a material that is suitable for the purpose of attaching the ultrasound system 1 and in particular the transducer array 3 to a patient's skin. Therefore, biocompatible and/or breathable materials may be used. The adhesive layer on the patch 52 is used to attach the patch 52 to the body and to make sure that the position of the transducer array 3 is essentially constant with respect to the patient's skin.

Additionally, or alternatively, the transducer array 3 and the TFT array 5 may be integrated into a strap 52, together with other wearable parts of the system.

Turning now to the front-end block 10 of the ultrasound system 1, this functional block is provided for the analog and digital control of the electronics.

In particular, the switch driver module 6, the receiver module 7 and the pulser module 8 of the front-end block10 are coupled with the transducer array 3 and the TFT array 5 for providing options such as control and read-out for the transducer array 3 and/or the TFT array 5. To this end, both data and electrical power may be transferred over the coupling between the modules 6, 7, 8 and the transducer array 3 and TFT array 5 enclosed within the adhesive patch 52.

Here, the switch driver module 6 is a TFT driver module, which is configured to provide a low-level interface to control and manage the TFT array 5 of the sensor array 2 and its individual TFT elements, respectively. In particular, the TFT driver module 6 of this embodiment can be integrated together with send and receive modules for controlling the generation of ultrasound pulses and detecting ultrasound signals, respectively. All these functional modules form part of the front-end block 10.

The receiver module 7 of the front end block 10 is configured to convert an analog input signal to a digital output signal.

The pulser module 8 of the front end block 10 is configured to control the transducer array 3 such that ultrasound pulses are generated. In particular, a beam-forming is performed here. Delay values may be set individually for each pulser unit of the system 1 that generates an ultrasound pulse. The delays on the different channels, in particular each pMUT element 3a, of the pulser unit will determine the focusing and the beam profile.

The digital middle-end block 11 of the ultrasound system 1 is configured to perform part of the control operations for the ultrasound system 1, and specifically to control the activity of the front-end block 10, the transducer array 3 and the TFT array 5 for generating ultrasound pulses and detecting ultrasound signals. The digital middle-end block 11 also functions to perform signal processing on the signals received from the front-end block 10.

As part of the digital middle-end block 11, microprocessors may be provided and/or other units for performing operations for controlling the ultrasound system 1 and its electronic components.

Thus, the ultrasound system 1 of the embodiment provides an easily customized ultrasound sensor and monitoring device.

The interface module may provide the possibility to build up a connection based on a wire connection and/or a wireless connection. In the present embodiment, examples for connection methods include USB and Ethernet as well as via Bluetooth and WiFi networks. The interface module may be configured to allow for data communication and/or power connections with external units.

In the embodiment, the PCB with its components and the TFT array 5 constitute an integrated control unit and data processing unit. PCB and TFT array 5, and the transducer array 3, may in different embodiments, be configured as units that are formed separately or they may be incorporated in a common unit.

In a further embodiment, the digital back-end block 12 may be implemented as a PC, directly on the embedded compute system or remotely on e.g. a cloud-based unit, which may be accessed through the interface module, either directly or indirectly via another device, such as a computer that is connected to a local or wide area network. The digital back-end block 12 may comprise processing algorithms (such as ultrasound image reconstruction algorithms, data analysis algorithms, visualization and control algorithms) for performing operations such as to reading and storing data, data analysis, configuration of the device, providing further information and administrating access the acquired data based on an authentication system.

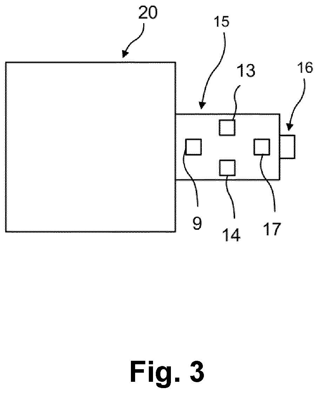

Turning to **Fig. 3****,** an embodiment of the ultrasound system 1 is exemplarily described. Reference is made to the embodiments of the ultrasound system 1 as described above. Figures 1 and 3 are meant to present analog embodiments of the system, although different aspects are highlighted and different elements are explicitly shown.

In **Fig. 3****,** a PCB 15 is shown that is connected to a sensor assembly 20. The sensor assembly 20 is comprised of the sensor array 2, interconnects (not shown) and may also contain the front-end ASIC's (not shown). The transducer array 3 and the TFT array 5 are not shown, since they are also integrated with the patch case 52.

The PCB 15 and sensor assembly 20 may be integrated in a patch case 52 (not shown) or alternatively in a strap 52. When integrated in a patch 52 or strap 52 the PCB 15 may be mounted onto the back of the sensor assembly 20, alternatively the PCB 15 may also be integrated as a separate assembly next to the sensor. Also, an interface module 16 is directly connected to the PCB 15.

The interface module 16 provides the possibility to build up a connection based on a wire connection and/or a wireless connection. Examples for connection methods include USB and Ethernet as well as via Bluetooth and WiFi networks.

The interface module 16 is configured to allow for data communication and/or power connections with external units.

In the embodiment shown in Fig. 3, circuits for controlling the ultrasound system 1 are incorporated on the PCB 15.

For instance, a compute unit 9 is provided on the PCB 15.

Also, a further interface control module 17 is provided and implemented with another IC. It provides a low-level interface to the interface module 16, thereby allowing to control the interface module 11.

Also, a memory module 13 is provided, herein specifically configured as a random access memory (RAM) module 13.

Also, a power management module 14 is provided.

In another embodiment, a power source may be provided, for example a battery. In this case, access to the stored power is provided to the system components via the power management module 14. In this case, the interface module 16 may be configured to provide a wireless data connection, in particular to a control unit.

In the present embodiment, another way of providing power to the system may be used, such as a connection to an external power source or an external power grid.

The compute unit 9, the interface module 16, the memory module 13 and the power management module 14 may be implemented in different ways, some of which may be known in the art. For example, a microprocessor, an ASIC chip and/or other units may be used.

The functionality of the front-end block 10, can be partially or completely implemented in one or multiple front-end ASICs, which may be integrated on the PCB 15 or together with the sensor array 2.

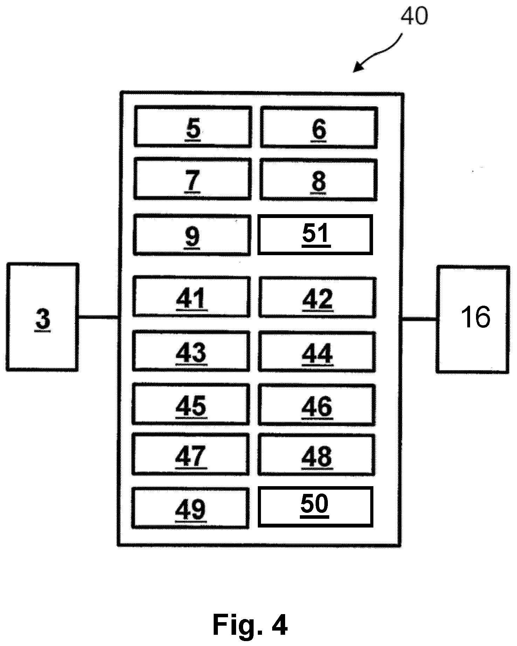

Turning to **Fig. 4****,** a schematic view of another embodiment is shown. The shown components may also be present in the embodiments described above; similar or equivalent elements have the same reference numerals and are not described again in detail.

Some or all of the elements and modules shown in **Fig. 4** may be implemented in different units, such as separately formed devices, or they may be implemented by way of program modules within the same computational unit and/or a digital memory device.

The combination of elements and modules of this embodiment is to be understood as exemplary, and as comprising a large number of optional features, which are not necessarily linked to each other or to be combined in one embodiment of the invention.

In **Fig. 4****,** the transducer array 3 is shown, which is linked to a control unit 40 (controlling the transducer array 3), which may be configured at least partially on a PCB 15 (e.g., on the PCB 15 of Fig. 3).

The connection between the transducer array 3 and the control unit 40 is a direct and cable-bound connection. However, in an alternative embodiment, an indirect and/or wireless connection is possible.

For instance, the control unit 40 can be attached to the patch 52 or patch case 52 (possible for wireless patches and wired patches). Further, there can be an additional control unit in a separate computer, in particular for the wired version.

The patch 52 is, for example, a 10cm by 10cm patch 52. The patch 52is intended to be placed in alignment with a selected anatomical landmark of the subject/the patient, which could be, for example, a given intercostal spacing, the sternum and/or any other feature easy to locate by the user/operator of the system 1, e.g., the operating physician. In particular, said landmark may be a rib, e.g., the 4^{th} rib, and/or the sternum, e.g., the top right left with respect to the center of the sternum (e.g., seen from a frontal view). The described exemplary landmark may be particularly advantageous for the left ventricle LV.

The transducer array 3, configured for providing ultrasound waves and/or receiving ultrasound signals, comprises a plurality of transducer array elements 3a.

A plurality of sub-arrays 4 are defined as a collection of one or more transducer array elements 3a of the plurality of transducer array elements 3a and are controlled as functional units for sending ultrasound pulses and/or receiving ultrasound signals. In this respect, a sub-array 4 is defined as an Ns by Ns (Ns x Ns) array of transducer array elements 3a / sensors, that act together for purposes of beamforming and image formation. The transducer array 3 is made of an array of N by N (N x N) transducer array elements 3a / sensors, wherein N >> Ns.

The sub-arrays 4 can be electronically activated such that any possible collection of adjacent Ns x Ns transducer array elements 3a / sensors can be used for the purposes of beamforming and image formation. Each sub-array 4 can perform pulse-echo measurements. Because there is the possibility of beamforming, these pulse-echo measurements can be steered in a finite collection of angles/directions. The measurement by an active sub-array 4 performing pulse-echo measurements on one single angle/direction can be referred to as a shot.

The control unit 40 comprises the TFT array 5, which was already described above.

The control unit 40 further comprises a dedicated data processing unit 41, which may in different embodiments comprise some of the other modules (as specified below with reference numerals 41 to 51) or which may in different embodiments be implemented by several individual modules. The data processing unit 41 receives and processes data from the transducer array 3.

Furthermore, the control unit 40 comprises a scan sequence module 42.

The scan sequence module 42 is configured for performing a scan sequence step, and the scan sequence module 42 is further configured to activate a sequence of sub-arrays 4, during the scan sequence step, for gathering ultrasound data.

A scan sequence is a collection of active sub-arrays 4 or shots, with or without an activation order.

Due to activating a sequence of sub-arrays 4 being defined by one or more transducer array elements 3a, ultrasound data may be gathered in a more goal-oriented/target-oriented manner, i.e., only the most relevant data may be gathered, thereby allowing the ultrasound system 1 to be operated in a more economical manner, in particular with respect to power consumption, as well as allowing the accuracy of the obtained ultrasound images to be increased.

In the scan sequence step, at least one scanning for gathering ultrasound data of at least one region of interest based on an activation of a sequence of sub-arrays according to a rank-order of the sub-arrays is performed / performable. In other words, a rank-order of all the possible sub-arrays/shots are generated and the sub-arrays/shots are chosen according to this rank order for gathering ultrasound data. This choice of the sub-arrays/shots (i.e., according to the rank-order) can characterize all features of the feature (e.g., tissue) of interest in the best possible way.

A rank-order in this context may refer to a ranking of all sub-arrays or shots in terms of how much they contribute to the characterization of the feature of interest. The ranking may be calculated with a weighted average of coverage and regularity. Sub-arrays/shots being at the top of the ranking may be chosen first. The rank-order may be influenced by previous choices of shots/sub-arrays, such that every time a sub-array/shot is chosen, the ranking needs to be re-calculated.

Prior to scanning, the transducer array 3 / the patch 52is placed on the subject body with a fixed relative position between it and the feature of interest FOI; and a fixed relative position between it and any blocking tissue that may interfere with the view of the feature of interest. Given these relative positions (e.g., feature of interest/patch/blocking tissue), it may be necessary to, firstly, place the patch 52 in a position that will maximize access of the signals to the feature of interest, i.e., the tissue of interest, and, secondly, rank-order all possible sub-arrays 4 or shots such as to acquire the most relevant data. This rank-order may consider a measure of quality of the data acquired by the sub-arrays 4 or shots and whether all parts of the feature of interest FOI are covered.

The rank-order of the sub-arrays 4 has a rank-order limitation.

In particular, a total budget of S sub-arrays 4/shots can be given, wherein the scan sequence module 42 chooses which are the best S sub-arrays 4/shots (out of a much large number of possible sub-arrays 4/shots). Hence, the scan sequence module 42 chooses a scan sequence for gathering ultrasound data. This choice of the best S sub-arrays 4/shots may characterize all features of the tissue of interest in the best possible way.

In the scan sequence step, the rank-order of the sub-arrays 4 is determined / determinable based on a coverage information and a regularity information achievable by the sub-arrays 4, when operated.

In other words, all sub-array positions are rank-ordered according to this criterium (i.e., the coverage and the regularity) and the first sub-array 4 is chosen. Then all remaining sub-arrays 4 are ranked ordered according to said criterium (considering that the first sub-array 4 has already been chosen) and the second sub-array 4 is chosen. The procedure continues until S sub-arrays 4 have been chosen.

In particular, as to the regularity of the shots, regularly spaced shots tend to produce better images that are easier to interpolate.

In one embodiment, e.g., the present embodiment, the regularity may be (e.g., is) defined as the average distance between all points in the tissue of interest, i.e., the feature of interest FOI, and a specific shot.

As to the coverage, the coverage is defined as the ratio of length/area/volume the tissue of interest, i.e., the feature of interest FOI, that can be observed to the total length/area/volume of the feature (e.g., the tissue) of interest.

In one embodiment, the coverage may be defined as the area/volume of the convex hull formed by the intersection of shots origination from all sub-arrays 4 in the scan sequence and the tissue of interest, i.e., the feature of interest FOI. The convex hull is a collection of points which the smallest convex set that includes all points of the feature of interest FOI.

The coverage information and the regularity information are obtainable by at least one initial scout scanning for gathering ultrasound data and/or by predetermined topography and/or location information. In the particular, said metrics (i.e., the coverage and the regularity) may require prior knowledge of the features inside the body before calculation; hence, said initial scout scanning may be performed for obtaining this knowledge.

In this respect, optionally, the initial scout scanning and/or the scanning according to the rank-order can additionally be based on general stochastic or quasi-stochastic information about a location of the region of interest ROI. In other words, the shot locations may be selected based on a stochastic or quasi-stochastic process.

Additionally, or alternatively, the scan sequence step may comprise at least one initial scout scanning for gathering ultrasound data for detecting at least one region of interest ROI, and at least one subsequent main scanning for only gathering ultrasound data of the region of interest ROI. In other words, due to performing an initial "rough" scouting scan, general regions of interest ROI may be detected, which may then be scanned in a more detailed manner, i.e., the scouting scan may be followed by a more detailed scan of the region of interest ROI. In particular, the detailed scan may be personalized towards the specific body topography of subject, i.e., the individual patient.

Additionally, or alternatively, the scan sequence step may comprise at least one initial scanning for gathering ultrasound data for detecting at least one region of interest ROI based on general stochastic or quasi-stochastic information about a location of the region of interest, and at least one subsequent main scanning for gathering ultrasound data of the region of interest ROI. In other words, the shot locations may be selected based on a stochastic or quasi-stochastic process. Due to the stochastic or quasi-stochastic nature of the process an initial "noisy" or "grainy" image may be produced, which may however already be used to detect general regions of interest ROI. Then a more detailed scan of the region of interest ROI may be performed. Furthermore, for example, in an iterative process said initial "noisy" or "grainy" image may be refined and the analysis repeated every "N" step, until a threshold is reached in a/the feature detection module 44 (which is described in more detail further below), e.g., based on convergence, number of iterations and/or another pre-defined metric.

Moreover, the control unit 40 comprises a reference module 43 and a feature detection module 44.

The reference module 43 is configured for obtaining reference ultrasound data.

The reference ultrasound data is obtained from the ultrasound data gathered by the scan sequence module 42.

The feature detection module 44 is configured for interfacing with the reference module 43 and for performing a determination step of the transducer array data gathered by the scan sequence module 42 to detect at least one feature of interest FOI.

The scan sequence module 42 is configured to interface with the feature detection module 44 and/or the reference module 43 for providing said ultrasound data to the feature detection module 44 and/or the reference module 43.

Alternatively or additionally, the scan sequence module 42 may be configured to interface with the feature detection module 44 and/or the reference module 43 for providing a scan sequence obtained by the scan sequence step to the feature detection module 44 and/or the reference module 43, to thereby allow the feature detection module 44 to perform a determination step for detecting at least one feature of interest and/or to thereby allow the reference module 43 to obtain the reference ultrasound data respectively based on the ultrasound data obtainable by scanning on the basis of said scan sequence. In other words, the scan sequence module 42 may provide a/the scanning sequence, which may be used to gather ultrasound data useable as input for either the reference module 43 and/or the feature detection module 44.

The feature detection module 44 is configured to provide at least one detection information to the reference module 43 determined by the determination step.

The feature detection module 44 is configured to, based on the detection information, establish and provide the reference ultrasound data to the reference module 43.

Additionally, or alternatively, the feature detection module 44 is configured to, based on the detection information, provide at least one region of interest ROI of the at least one feature of interest FOI to the reference module 43, to thereby allow the reference module 43 to subsequently establish the reference ultrasound data (itself, e.g., by means of a detail scanning, e.g., in accordance to the rank-order).

Additionally, or alternatively, at least one region of interest of the at least one feature of interest may be supplied by the feature detection module 44 directly to the scan sequence module 42, which can execute a patient specific/optimized scan sequence and provide the measured data to the reference module 43 to subsequently establish the reference ultrasound data.

In particular, when the transducer array 3 is placed on the body (e.g. the thorax) of the subject/patient, it does not have any prior information on the features of the body on which it is placed. Thus, after an initial scout scanning, the coverage and the regularity information are determined, e.g., by the control unit 40 and/or the data processing module 41, to thereby allow the scan sequence module 42 to perform the scan sequence step.

In the scan sequence step, an activation of a/sequence of sub-arrays 4 is performed for gathering ultrasound data (on the basis of the above-described rank-order).

The obtained ultrasound data can be provided to the feature detection module 44 which can analyze the characteristics of the received ultrasound signals or the image data derived from these ultrasound signals, i.e., the transducer array data, for detecting the features of interest FOI and generate the detection information.

Subsequently, when the results satisfy a predetermined requirement, the feature detection module 44 establishes and provides the reference ultrasound data to the reference module 43.

The predetermined requirement can be a certain resolution quality, i.e., a high resolution, in particular a resolution being sufficiently high for further processing.

Alternatively, or additionally, when the results do not satisfy a predetermined requirement, e.g., said high resolution, the feature detection module 44 provides at least one region of interest ROI of the at least one feature of interest FOI to the reference module 43.

The reference module 43 can then provide a detailed scanning of said region ROI, e.g., also on the basis of the above-described rank-order determined in the scan sequence step.

Hence, this scanning will allow to detect the feature of interest/target FOI, and, accordingly, reference ultrasound data may be obtained (e.g., by the reference module 43 itself).

The determination step comprises
- determining and extracting relevant feature points, in particular relevant feature points with respect to the feature of interest FOI, from the transducer array data by phase-based and/or morphological image processing, wherein the transducer array data are receivable by the feature detection module 44. The ultrasound data being recorded when an ultrasound pulse is generated by at least one transducer array element 3a and an ultrasound signal is subsequently received by the same and/or another transducer array element 3a (i.e., generated and received by sub-arrays 4 in accordance to the scan sequence, i.e., the rank-order),
- aligning the relevant feature points with at least one predetermined information representing a geometrical model GM of the feature of interest FOI, and
- evaluating the match between the relevant feature points and the at least one predetermined information, to thereby generate the detection information comprising the feature of interest FOI and its associated region of interest ROI.

The at least one predetermined information comprises at least one simple geometrical shape, such as an ellipsoid, and/or at least one template model derived from one or more predetermined features of interest of one or more subjects and/or at least one template model being based on statistical shape analysis of a predetermined population (i.e., larger group of subjects sharing at least one mutual characteristic, e.g., age, gender, etc.).

In this respect, the at least one predetermined information comprises myocardial tissue information and/or myocardial tissue interface information, in particular the at least one predetermined information may comprise left ventricle information.

The determination step further comprises, before aligning, skeletonizing the relevant feature points for reducing the number of feature points. Hence, calculations can be speeded up and, accordingly, processing time can be brought to a time frame, which, e.g., may be compatible with real-time use on monitoring applications such as the present ultrasound system 1, since, instead of aligning all the feature points, i.e., the full point cloud, of the detected features, pre-processing may be performed using a skeletonization operator, i.e., skeletonizing may be performed, that reduces the number of feature points in the target feature shape cloud while keeping good alignment accuracy.

In the determination step, the feature detection module 44 can additionally determine whether the feature of interest FOI is located in the region of interest ROI.

Hence, feedback information is generatable for providing a basis for a critical user feedback, thereby allowing a/the user to correct the positional placement of the transducer array 3 / the patch 52 for gaining better and/or more complete ultrasound imaging results of the targeted features, i.e., the features of interest FOI.

The control unit 40 may further comprise a feedback module (not shown in Fig. 4) configured for providing a request for repositioning the transducer array 3 based on the determination step.

In one embodiment of the determination step, alternatively or additionally, in the determining and extracting relevant feature points, features from the acquired transducer array data may be extracted to locate relevant points of high probability of belonging to myocardial tissue and/or its interface with blood pools in the heart. This process may use phase-based image processing and/or other relevant algorithms, including but not limited to morphological operations to spatially re-arrange the selected feature points. Optionally, said skeletonization may be applied to reduce the dimensionality of the cloud of selected feature points.

In one embodiment of the determination step, alternatively or additionally, in aligning the relevant feature points with at least one predetermined information, a relevant geometrical model for a selected anatomical target may be aligned with the selected feature points. In one embodiment, this anatomical target might be the left ventricle, but could be other cardiac chambers or structures in another embodiment. In one embodiment, the geometrical for the left ventricle might be a simple ellipsoid. In another embodiment, the geometrical for the left ventricle may be constructed via statistical shape modelling of a population of subjects/patients, from where the average of their left ventricles and their main modes of variation are extracted to estimate relevant geometrical models to detect on incoming ultrasound images. In an embodiment, the alignment between the cloud of the selected feature points may be done with point cloud registration algorithms.

Optionally, in order to further personalize the relevant geometrical model for the anatomical target may be refined to further match the subject-specific/patient-specific anatomy of the subject/patient wearing the transducer array 3 / ultrasound patch 52. In one embodiment, this refinement may be done with real-time segmentation via B-spline Explicit Active Surfaces, using the same features originally detected in determining and extracting relevant feature points to extract the selected feature points.

Accordingly, in one further embodiment of the determination step,
- the determining and extracting relevant feature points further comprises:
   - calculating contrast-insensitive features using monogenic signal theory applied to the transducer array data (e.g., to 3D ultrasound images), to thereby generate 3D feature maps, and
   - (subsequently) binarizing the feature maps to generate "thick" regions around candidate positions for the surface of the feature of interest FOI, in the present case, the left ventricle LV, which will serve as target positions;
- the determination step further comprises, before aligning, skeletonizing the binarized feature maps, to thereby yield a reduced size cloud of points in the vicinity of mid-myocardium and/or endo/epicardial left ventricle contours; and
- the aligning further comprises:
   - selecting an appropriate left ventricle LV template model (i.e., geometrical model) to be fit to the image data, i.e., transducer array data, wherein the template model ranges from a simple ellipsoid to a realistic left ventricle surface extracted from other patients and/or from statistical shape analysis from a population, and
   - (subsequently) performing the spatial alignment between the target point cloud of detected features and the selected left ventricle template model, wherein this alignment may be robustly done using existing algorithms such as Coherent Point Drift or Finite Iterative Closest Point, and
   - optionally, refining the aligned left ventricle template model using image segmentation (e.g. BEAS).

As already outlined above, since the feature detection module 44 can determine whether the feature of interest FOI is located in the region of interest ROI the system 1 and due to the feedback module, a/the user may be requested to reposition the transducer array 3, i.e., the patch 52, on the body of the subject to improve the visibility of the feature of interest FOI, in order to alter the geometrical relation between the transducer array 3, i.e., the patch52, and the position of the feature of interest FOI.

In this respect, evaluating the match of the determination step further comprises evaluating whether the feature of interest FOI is completely represented by the relevant feature points, to thereby determine whether the feature of interest FOI is located in the region of interest ROI.

Additionally, evaluating the match of the determination step also comprises determining a region of the region of interest ROI, where relevant feature points are missing, based on the at least one predetermined information.

Optionally, evaluating can further comprise detecting parts in the transducer array data being occluded by the presence of different features, such as rib/bone/lungs. Hereto, the existence of sufficient image contrast may be estimated by taking the value of the dynamic range of the transducer array data (e.g., at depths beyond 6 cm). Automatic discarding of the occluded regions may be done by setting an automatic threshold, which may be estimated via Otsu's method. To estimate this, dynamic range values of the images based on the transducer array data taken over the entire extent of the transducer array may use to feed the Otsu's method to ensure that both non-occluded and occluded regions are considered for the estimation of the threshold value.

Also, the control unit 40 comprises a volume reconstruction module 45 being configured, in particular, for reconstructing the volume of a target organ or part of a target organ, which is in this embodiment the left ventricle LV of a subject equipped with the ultrasound system 1.

The volume reconstruction module 45 can reconstruct the volume of the target organ/feature of interest FOI, e.g., the left ventricle LV, based on the reference ultrasound data obtained by the reference module 43.

The target organ or the part of the target organ can be considered as a/the feature of interest FOI, and vice versa. In other words, the feature of interest FOI is a target tissue or anatomical structure and/or part of a target tissue or anatomical structure. For example, the target organ/the feature of interest FOI can be the heart, left/right ventricles, left/right atria, blood vessels, aorta, lungs, etc.

Furthermore, the control unit 40 comprises a selected monitoring unit 46 being configured, in particular, for monitoring the target organ or part of the target organ, e.g., the left ventricle LV (e.g., by means of the transducer array 3), which, in the present embodiment, is the feature of interest FOI.

Central to the selected monitoring unit 46 may be the fact that not the entire volume of the feature (i.e., target organ or part of a target organ) is traced, and not the entire transducer array 3 is used (thus not all transducer elements 3a are used for tracing). In this respect, the selected monitoring unit 46 may be configured for selective monitoring based on the feature of interest FOI detected in the determination step.

The control unit 40 may optionally comprise one or more additional modules for other purposes. For example, in case of the embodiment shown in Fig. 4, the control unit 40 also comprises a correction module 47, a complexity reduction module 48, a deformation correction module 49, an artifact determination module 50, and a phase determination module 51. However, the system 1 and/or, more specifically, the control unit 40 may optionally comprise only one of these modules 47 to 51, or more, wherein these modules 47 to 51 may be comprised in the system 1 and/or, more specifically, in the control unit 40 in any combination.

In this respect, the correction module 47 is configured for correction of effects in analog ultrasound signals caused by bending of the flexible transducer array 3.

The complexity reduction module 48 is configured for computational operations that implement a function of complexity reduction (e.g., combing transducer array elements 3a, forming sub-arrays 4, and/or goal-oriented controlling of transducer array elements 3a). Hence, the complexity reduction module 48 may be configured to define at least one sub-array 4 of transducer array elements 3a and to control the at least one sub-array 4 as a functional unit for sending at least one ultrasound pulse and/or receiving at least one ultrasound signal.

The deformation correction module 49 is configured to perform a deformation correction step, wherein deformation information is determined for the transducer array 3.

The artifact determination module 50 is configured for identification of artifacts and avoiding obstructions to ultrasound imaging caused by artifacts, such as air bubbles between the transducer array 3 and the skin of the subject, e.g., the patient, equipped with the system 1 or bones, e.g. ribs. In particular, strong ultrasound reflection of the ribs may be used for identification, as well as the dropout of echoes coming from under the ribs.

The phase determination module 51 is configured for determining cardiac cycle phases in particular end-systolic phase and end-diastolic phase, as for evaluation of the stroke volume only the end-diastolic and end-systolic phases are of importance. In this context, a detailed volume scanning at the end-diastolic and end-systolic phase may be enabled by means of the selected monitoring unit 46, which may be correspondingly configured.

Each of said modules 41 to 51 may be implemented as a software module, comprising instructions for computational operations that implement their respective function. Such a software module may in particular be implemented together with the control unit 40.

Specifically, each of said modules 41 to 51 may be comprised, at least partially, by the control unit 40 and/or by the data processing unit 41.

The system 1 according to the embodiment is controlled by integrated support electronics. The support electronics comprise ASIC elements distributed along the periphery of the sensor. The sensor may comprise the transducer array 3. The ASIC elements could also be located on the PCB 15.

Also, the rest of the electronics is integrated on the, e.g., flexible, PCB 15, which is in turn connected to the sensor.

The ASIC elements are distributed along the edge of the flexible ultrasound sensor and have some or all of the following functions:
- Actuating the mechanical transducers, i.e., the transducer array elements 3a;
- Processing and digitizing signals received by the transducer array elements 3a;
- Controlling the switching of the TFT array 5.

The flexible PCB 15 also comprises a digital "master" ASIC 9 and/or a microprocessor, for performing digital signal processing, and control of the other ASIC elements. The digital signal processing and/or control may also be implemented in part or completely on the ASICs distributed along the periphery and/or edge of the sensor (e.g. routed to the backside of the sensor on a PCB, e.g., the PCB 15, by means of interconnects).

In particular, the ultrasound system 1 is operating as follows:
The ultrasound system 1 has the patch 52 and/or strap 52with transducer array 3.

Once the patch 52 has been applied to the body it is not yet "aware" of the features present inside the body. A measurement needs to establish which features are present within the field of view. At the highest level this consists of two steps:
- Step (a): Performing pulse-echo measurement inside the field of view of the sensor (i.e. the transducer array 3), i.e. executing a scan sequence;
- Step (b): Analyzing the measurement data to detect the features of interest. This second step will be performed by the feature detection module.

When performing the scan sequence step (a), in principle the topology (or physiological features) inside the field of view are still unknown. Therefore, a more detailed scanning would be needed, to establish a good 3D structure of all the features present inside the field of view. However, a detailed 3D scan would require a lot of resources to perform (i.e. long acquisition time, high power, bandwidth, memory, ....) and would not be energy efficient. Therefore, a method would be required to perform the scan sequence in an efficient manner, while still allowing the feature detection module to identify the relevant physiological regions of interest.

In this disclosure a "scan sequence module" is described that performs the scan sequence in an efficient manner.

For the patch 52 of the ultrasound system 1, a sub-array 4 is defined an an Ns x Ns array of sensors (i.e. the transducers 3) that act together for purposes of beamforming and image formation. The patch 52 is made of an array of N x N sensors, where N >> Ns.

Sub-arrays 4 can be electronically activated such that any possible collection of adjacent Ns x Ns sensors can be used for the purposes of beamforming and image formation. Each sub-array can perform pulse-echo measurements. Because there is possibility of beamforming, these pulse-echo measurements can be steered in a finite collection of angles/directions. An active sub-array 4 can perform pulse-echo measurements on one single angle/direction, this measurement is referred to as a shot.

A scan sequence is a collection of shots, with or without an activation order.

The patch 52 is placed on the body with a fixed relative position between it and the tissue of interest; and a fixed relative position between it and any blocking tissue that interferes with the view of the tissue of interest. Given that these relative positions path/tissue of interest and patch/blocking tissue, it is necessary to 1) place the patch 52 in a position that will maximize access of the signals to the tissue of interest and 2) rank order all possible sub-arrays or shots such as acquire the most relevant data. This rank-order considers a measure of quality of the data acquired by the sub-arrays or shots and whether all parts of the tissue of interest are covered.

The scan sequence module 42 is a combination of hardware control logic for selecting sub-array positions/shots and software modules that implement the scanning sequences. To assure a good field of view of the region of interest for the scan sequence module, a procedure can be included for patch 52 placement that can help to maximize the chances of having a good field of view on the region of interest, i.e. a clear external body reference for placing the patch 52 for cardiac monitoring can be provided as part of the procedure.

The scan sequence is a sequence of sub-arrays or pulse-echo measurements (also called shots) meant to scan/measure/image the region of interest. In general terms the scan sequence can refer to any activation sequence of transducers, during send and receive, with the purpose of gathering the measurement data that is used for establishing a 3D/2D or 1D image of the region of interest.

A typical scan sequence could be a regular grid of sub-array positions, where for each sub-array position a number of measurements is performed for different "beam-directions". This can be done by measuring all possible positions or by performing a sparser stepping of the possible positions. This might lead to suboptimal usage of the available resource as many of these measurements would contain area's which are not necessarily related to the area of interest.

Smarter scan sequences can therefore be integrated in the scan sequence module 42, which can lead to a more efficient scanning of the region of interest or provide a good distribution of measurements over the region of interest with minimal amount of shots.

Some specific examples are:
a) Performing in initial "rough" scouting scan meant to detect general regions of interest. This can then be followed by a more detailed scan of the region of interest. In essence the detailed scan is personalized towards the specific body topography of the individual patient.
b) Stochastic or quasi-stochastic imaging, where the shot locations are selected based on a stochastic or quasi-stochastic process. Due to the stochastic or quasi-stochastic nature of the process an initial "noisy" or "grainy" image will produced, which can however already be used to detect general regions of interest. In an iterative process this image can be refined and the analysis repeated every "N" step, until a threshold is reached in the feature detection module (e.g. based on convergence, number of iterations or another pre-defined metric).
c) Determining the scan sequence based on an algorithm to compute a rank-order of all the possible sub-array/shots and choose them according to this rank order. Given a total budget of S sub-arrays/shots, the algorithm chooses which are the best S sub-array/shots (out of a much large number of possible sub-arrays/shots)-i.e. the algorithm chooses a scan sequence. This choice of the best S sub-arrays/shots characterizes all features of the tissue of interest in the best possible way. An example of such a ranking algorithm comprises two metrics. The first metric is the regularity of the shots. Regularly spaced shots tend to produce better images that are easier to interpolate. The second metric is the coverage. Coverage is defined as the ratio of the length/area/volume the tissue of interest that can be observed to the total length/area volume of the tissue of interest.

In principle point a) can be combined with b) and/or c), e.g. the scouting scan can be a stochastic or quasi-stochastic imaging scan followed by a detailed scanning based on optimal shot locations; both scouting scan and detailed scan can be based on stochastic or quasi-stochastic imaging; or any other combination thereof.

For point c), the calculation of the metrics requires prior knowledge of the features inside the body. This knowledge can be obtained by:
1) The scouting scan from point a.
2) This optimal shot positions can be precomputed, based on a large sample of patient body topologies and organ locations.

The scan sequence module 42 according to the present disclosure can work in collaboration with the rest of the ultrasound system 1, where the scan sequence module 42 provides the scanning routine, which can be used to gather data useable as input for either the reference module or the feature detection module.

The present disclosure and in particular scan sequence module 42 is of high importance for power management. Each activation of a sub-array has a cost in power. It is important to try to minimize the number of active sub-arrays while maintaining good image quality.

The present disclosure is of part of the optimization/personalization of the scan sequence. With time, the ultrasound system 1 with its patch 52 learns how to best acquire the most relevant data. This help to save power and increases accuracy of the images.

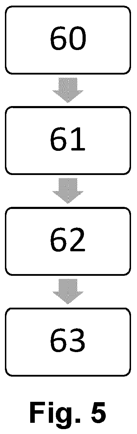

Turning to **Fig. 5****,** a method for operating the ultrasound system 1 is described. The following description is based on the embodiment of the ultrasound system 1 described above with reference to, e.g., **Fig. 4****,** but other embodiments of the ultrasound system 1 may be suited as well.

The method for ultrasound imaging of a body part of a subject comprising at least the following steps:
- in step 60, placing a transducer array 3 with a plurality of transducer array elements 3a for providing ultrasound waves for transmission into the body of the subject, and
- in step 61, performing a scan sequence step for obtaining ultrasound data based on reception of the ultrasound waves transmitted into the body of the subject (e.g. by scanning).

The scan sequence step comprises at least one scanning for gathering ultrasound data of at least one region of interest ROI based on an activation of a sequence of sub-arrays 4 according to a rank-order of the sub-arrays 4.

The rank-order of the sub-arrays 4 has a rank-order limitation, which defines a limited rank-order of sub-arrays 4 representing a smaller number of sub-arrays 4 than the one of the rank-order of the sub-arrays 4.

In step 60, placing further comprises aligning the transducer array 3 based on at least one landmark of the body of the subject.

The landmark can be a rib and/or the sternum of the subject.

The method further comprises
- in step 62, performing a determination step of the obtained ultrasound data to detect at least one feature of interest FOI (in this embodiment, the left ventricle LV), and
- in step 63, obtaining reference ultrasound data based on at least one detection information determined by the determination step.

In particular, the method of Fig. 5 serves the purpose of operating the ultrasound system 1. Thus, it may have the same features and/or the same advantages as the ultrasound system 1 of the invention.

Regarding the scan sequence step (as well as the determination step), reference is particularly made to the description with respect to Fig. 4.

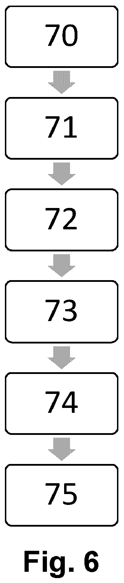

Turning to **Fig. 6****,** a further embodiment of a method for operating the ultrasound system 1 is described. The following description is based on the embodiment of the ultrasound system 1 described above with reference to, e.g., **Fig. 4****,** but other embodiments of the ultrasound system 1 may be suited as well.

In this respect, steps 70, 71, 72 and 73 are substantially the same as steps 60, 61, 62 and 63, respectively, wherein the method additionally comprises the following steps:
- in step 74, reconstructing the volume of the target tissue or anatomical structure and/or part of the target organ based on the reference ultrasound data (in this embodiment, the left ventricle LV), and
- in step 75, monitoring the volume of the target tissue or anatomical structure and/or part of the target tissue or anatomical structure.

In particular, the method of Fig. 6 serves the purpose of operating the ultrasound system 1. Thus, it may have the same features and/or the same advantages as the ultrasound system 1 of the invention.

Regarding the scan sequence step (as well as the determination step), reference is particularly made to the description with respect to Fig. 4.

In step 60 or step 70, placing the transducer array 3 can comprise placing an ultrasound patch 52 (e.g., a 10 cm by 10 cm patch 52) on top of the surface of a body part of a subject, e.g., the patient's chest.

In step 60 or step 70, placing the transducer array 3 can further comprise aligning the transducer array 3/the patch 52 with a selected anatomical landmark of the subject/the patient, which could be, for example, a given intercostal spacing, the sternum and/or any other feature easy to locate by the user/operator of the system 1, e.g., the operating physician.

A measuring mode of the ultrasound system is active at least between steps 60, 70 and 61, 71. Thus, the system 1 is configured to perform measurements using the transducer array 3 (e.g., in step 61, 71).

In step 62 or step 72, the method may further comprise providing a request for repositioning the transducer array 3 based on the determination step (e.g. by means of a feedback module). The request may be provided to the user/operator of the system 1, e.g., the operating physician, Hence, a feedback mechanism can thereby be provided to allow improving the visibility of the feature of interest FOI, e.g., the left ventricle LV, by altering the geometrical relation between the transducer array 3/the patch 2 and the position of the feature of interest FOI (based on said request for repositioning).

Referring to the above-mentioned, scanning may be based on ultrasound based pulsed echo measurements with focused beams, wherein ultrasound beams are beam-formed by selecting transducer array elements 3a of the transducer array 3 (e.g., a subset and/or a sub-array 4 within the transducer array elements 3a) and by correspondingly operating said transducer array elements 3a.

Note that the target organ (here the left ventricle LV) may in general comprise a heart or blood vessel and the part of the target organ may comprise a ventricle or atrium. In addition to targeting an organ, other volumetric features inside the body may be imaged, such as the volume of a blood inside the body due to e.g. internal hemorrhaging.

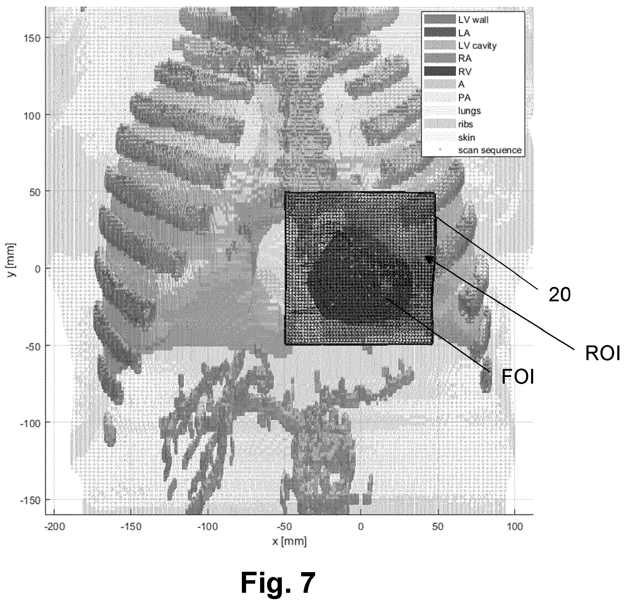

Turning to **Fig. 7****,** a visualization of an exemplary image obtained on the basis of the method of Fig. 5 is shown. In particular, an example on the basis of real CT data is shown. In the present case, blocking tissue are lungs and ribs. The feature, i.e., tissue, of interest is the left ventricle LV of the heart. The rank-order limitation, i.e., a sub-array budget, is 24 in the present case. However, any other rank-order limitation may be applicable. By selecting the sub-array positions with the proposed method (e.g., method of Fig. 5 or 6), 95 % of the feature of interest FOI is covered with less than 25 active sub-arrays. Selected sub-array 4 are shown as light-colored dots. This figure also illustrates the placing of the 10cm by 10 cm patch 52, with the top right tip aligned with the 4th rib.

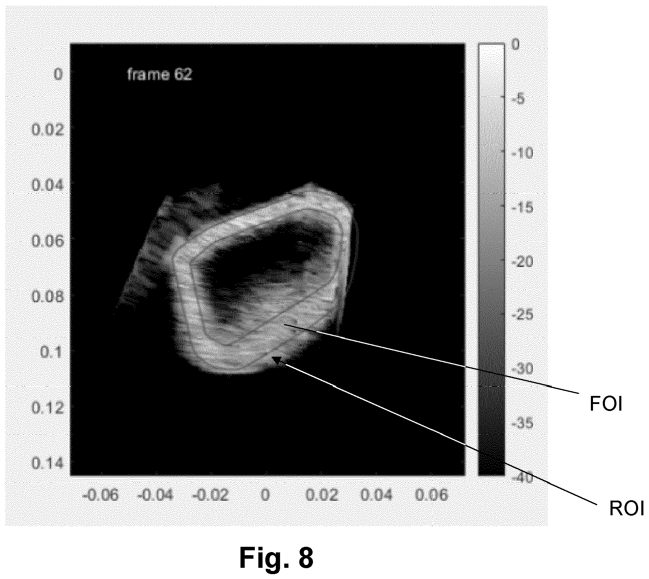

Turning to **Fig. 8****,** an example of an ultrasound simulation obtained from the image of Fig. 7 is shown. In other words, an example of an ultrasound simulation is shown. In particular, this is a horizontal slice of the 3D volume obtained from the example above. The selection of sub-arrays 4 (in accordance to the rank-order) creates a high-quality image.

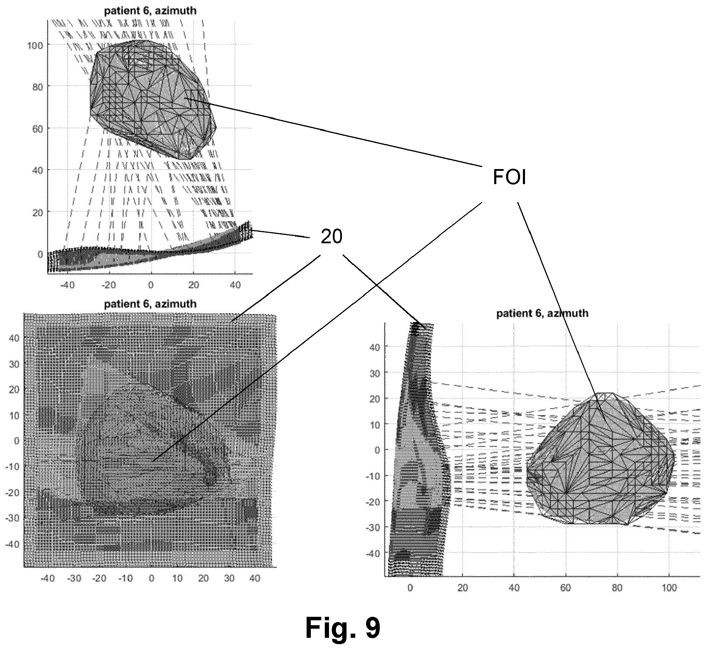

Turning to **Fig. 9****,** a visualization of an example of a sub-array selection based on the method of Fig. 5 is shown. Light colored dots denote unblocked sub-array positions. Dark colored dots denote blocked sub-array positions. Here, the frontal (bottom left figure), top (top left figure) and lateral (bottom right figure) views are shown respectively illustrating the chosen sub-arrays 4 and their respective shots. Coverage of the left ventricle LV is more than 95 % and, in particular, is accomplished with less than 50 shots.

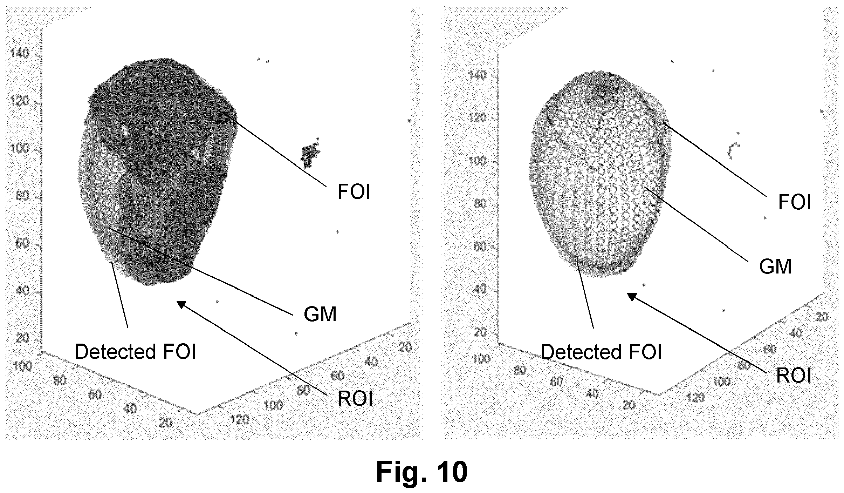

Turning to **Fig. 10****,** a visualization of a skeletonizing step of a determination step, which may be part of a method to operate the ultrasound system, is shown. The visualization of the skeletonizing step of Fig. 10 may refer and apply to any of the methods as already described above (e.g., with respect to Figures 4, 5 and/or 6), in which a determination step is performed by a feature detection module 44.

As can be seen in the left part of Fig. 10, the geometrical model GM, i.e., the left ventricle LV template, represented as an ellipsoid (cf. structure depicted with circular shapes), is fitted to the feature of interest FOI, i.e., in the present case, the contrast-insensitive features found in the 3D ultrasound image (cf. dark colored structure). Furthermore, the detected feature of interest FOI can also be seen (cf. light colored structure).

Further, as can be seen in the right part of Fig. 10, the same is shown as in the left part of Fig. 10, but a skeletonization step is applied to the 3D ultrasound image. Note that the fitted ellipsoid (cf. structure depicted with circular shapes) is very similar regardless the fact that the target point cloud is reduced by more than 10 times (cf. dark colored structure). Furthermore, the detected feature of interest FOI can also be seen (cf. light colored structure), which is substantially the same as shown in the left part of Fig. 10.

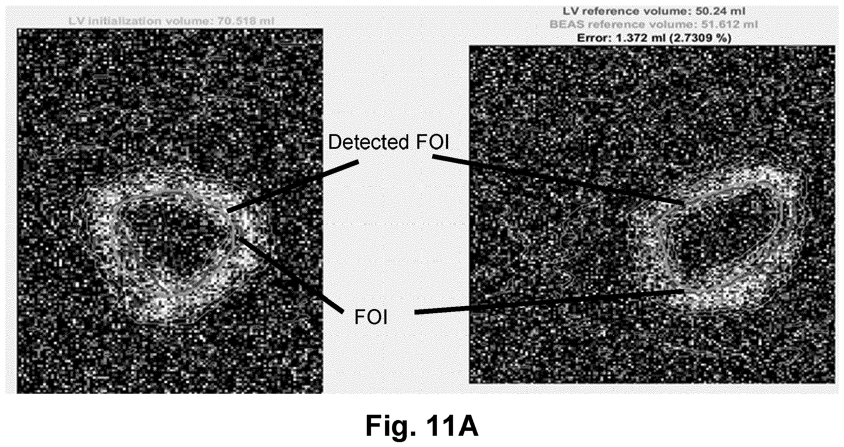

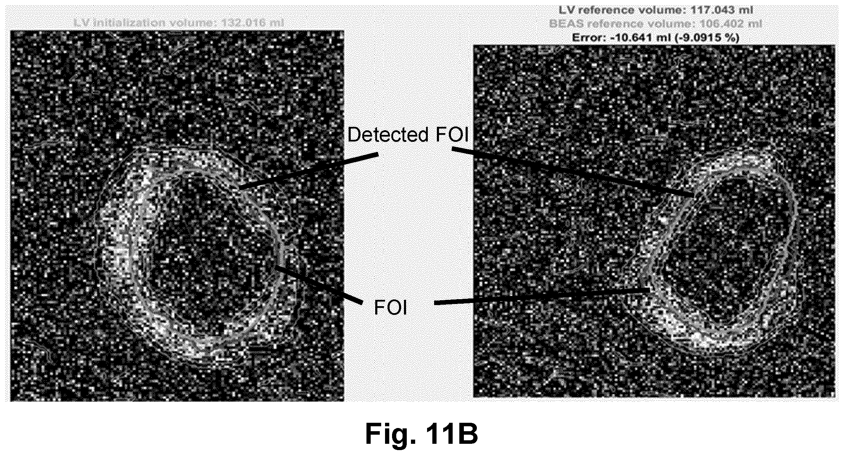

Turning to **Fig. 11A** and B, a visualization of a feature of interest detected on the basis of the disclosure with respect to Figs. 4, 5 and/or 6 is respectively shown.

In Fig. 11A, a detected left ventricle LV (cf. light colored line) on a noisy simulated ultrasound dataset (left and right correspond to XZ and YZ planes inside the 3D image) is shown. Note that the detected left ventricle LV is closely aligned with the true left ventricle LV shape (cf. dark colored line), both in terms of local position as well as in terms of overall object volume.

In Fig. 11B, another dataset is shown in the same manner as in Fig. 11A, wherein a detected left ventricle LV (cf. light colored line) on a noisy simulated ultrasound dataset (left and right correspond to XZ and YZ planes inside the 3D image) is shown.

The following aspects could also be part of the invention:
Aspect 1: Ultrasound system, in particular for cardiac monitoring, comprising
   - a transducer array with a plurality of transducer array elements,
   - a control unit, which is coupled to the transducer array, for controlling the transducer array, and
   - a data processing unit for receiving data from the transducer array and processing the received data,

   wherein the system further comprises
      - at least one reference module configured for obtaining and/or providing reference ultrasound data, and
      - at least one feature detection module configured for interfacing with the reference module and for performing a determination step of the transducer array data to detect at least one feature of interest (FOI),
   wherein the feature detection module is configured to provide at least one detection information to the reference module determined by the determination step.
Aspect 2: The ultrasound system according to aspect 1,
   **characterized in that**
   the feature detection module is configured to, based on the detection information, establish and provide the reference ultrasound data to the reference module and/or provide at least one region of interest (ROI) of the at least one feature of interest (FOI) to the reference module, to thereby allow the reference module to subsequently establish the reference ultrasound data.
Aspect 3: The ultrasound system according to aspect 1 or 2,
   **characterized in that**
   the feature detection module is further configured to, in the determination step, determine whether the feature of interest (FOI) is located in the region of interest (ROI).
Aspect 4: The ultrasound system according to one of the preceding aspects,
   **characterized in that** the system further comprises a feedback module configured for providing a request for repositioning the transducer array based on the determination step.
Aspect 5: The ultrasound system according to one of the preceding aspects,
   **characterized in that**
   the determination step comprises
   - determining and extracting relevant feature points from the transducer array data by phase-based and/or morphological image processing, wherein the transducer array data are receivable by the feature detection module, when an ultrasound pulse is generated by at least one transducer array element and an ultrasound signal is subsequently received by the same and/or another transducer array element,
   - aligning the relevant feature points with at least one predetermined information representing a geometrical model (GM) of the feature of interest (FOI),
   - evaluating the match between the relevant feature points and the at least one predetermined information, to thereby generate the detection information comprising the feature of interest (FOI) and its associated region of interest (ROI).
Aspect 6: The ultrasound system according to aspect 5,
   **characterized in that**
   the determination step further comprises, before aligning, skeletonizing the relevant feature points for reducing the number of feature points.
Aspect 7: The ultrasound system according to aspect 5 or 6,
   **characterized in that**
   the at least one predetermined information comprises myocardial tissue information and/or myocardial tissue interface information, in particular the at least one predetermined information comprises left ventricle information.
Aspect 8: The ultrasound system according to one of aspects 5 to 7,
   **characterized in that**
   evaluating the match of the determination step further comprises evaluating whether the feature of interest (FOI) is completely represented by the relevant feature points, to thereby determine whether the feature of interest (FOI) is located in the region of interest (ROI).
Aspect 9: The ultrasound system according to aspect 8,
   **characterized in that**
   evaluating the match of the determination step further comprises determining a region of the region of interest (ROI), where relevant feature points are missing, based on the at least one predetermined information.
Aspect 10: The ultrasound system according to one of the preceding aspects,
   **characterized in that**
   the system further comprises at least one volume reconstruction module configured for reconstructing the volume of a target organ or part of a target organ.
Aspect 11: The ultrasound system according to aspect 10,
   **characterized in that**
   the system further comprises at least one selected monitoring unit configured for monitoring the volume of the target organ and/or part of the target organ.
Aspect 12: Method for ultrasound imaging of a body part of a subject comprising at least the following steps:
   - placing a transducer array with a plurality of transducer array elements for providing ultrasound waves for transmission into the body of the subject,
   - obtaining transducer array data based on reception of the ultrasound waves transmitted into the body of the subject,
   - performing a determination step of the transducer array data to detect at least one feature of interest (FOI),
   - obtaining reference ultrasound data based on at least one detection information determined by the determination step.
Aspect 13: The method according to aspect 12,
   **characterized in that**
   the method further comprises providing a request for repositioning the transducer array based on the determination step.
Aspect 14: The method according to aspect 12 or 13,
   **characterized in that**
   the method further comprises reconstructing the volume of the target tissue or anatomical structure and/or part of the target organ.
Aspect 15: The method according to aspect 14,
   **characterized in that**
   the method further comprises monitoring the volume of the target tissue or anatomical structure and/or part of the target tissue or anatomical structure.

### Reference numerals

- 1: ultrasound system
- 2: sensor array
- 3: transducer array; pMUT array
- 3a: transducer array element; pMUT element; transducer array unit
- 3b: transducer array set
- 4: sub-array
- 5: switch array; thin-film transistor (TFT) array
- 6: switch driver module; TFT driver module
- 7: receiver module; analog-digital converter (ADC)
- 8: pulser module
- 9: digital application-specific integrated circuit (ASIC)
- 10: front-end block
- 11: digital middle-end block
- 12: back-end block
- 13: memory module (RAM)
- 14: power management module
- 15: PCB
- 16: interface module
- 17: interface control module; ASIC
- 20: sensor assembly
- 22: signals
- 40: control unit
- 41: data processing unit
- 42: scan sequence module
- 43: reference module
- 44: feature detection module
- 45: volume reconstruction module
- 46: monitoring unit
- 47: correction module
- 48: complexity reduction module
- 49: deformation correction module
- 50: artifact determination module
- 51: phase determination module
- 52: patch/strap
- 60: step
- 61: step
- 62: step
- 63: step
- 70: step
- 71: step
- 72: step
- 73: step
- 74: step
- 75: step
- FOI: feature of interest
- ROI: region of interest
- GM: geometrical model
- N: number of rows
- M: number of columns

## Claims

1. Ultrasound system (1), in particular for cardiac monitoring, comprising
- a transducer array (3) with a plurality of transducer array elements (3a),
- a control unit (40), which is coupled to the transducer array (3), for controlling the transducer array (3), and
- a data processing unit (41) for receiving data from the transducer array (3) and processing the received data,
wherein the system (1) further comprises
- a plurality of sub-arrays (4) defined as a collection of one or more transducer array elements (3a) and controlled as functional units for sending ultrasound pulses and/or receiving ultrasound signals, and
- at least one scan sequence module (42) configured for performing a scan sequence step, wherein the scan sequence module is configured to activate a sequence of sub-arrays (4), during the scan sequence step, for gathering ultrasound data.

2. The ultrasound system (1) according to claim 1,
**characterized in that**
the scan sequence step comprises at least one initial scout scanning for gathering ultrasound data for detecting at least one region of interest (ROI).

3. The ultrasound system (1) according to claim 1 or claim 2,
**characterized in that**
the scan sequence step comprises at least one subsequent main scanning for only gathering ultrasound data of the region of interest (ROI).

4. The ultrasound system (1) according to one of the preceding claims,
**characterized in that**
the scan sequence step comprises
- at least one initial scanning for gathering ultrasound data for detecting at least one region of interest (ROI) based on general stochastic or quasi-stochastic information about a location of the region of interest (ROI), and
- at least one subsequent main scanning for gathering ultrasound data of the region of interest (ROI).

5. The ultrasound system (1) according to one of the preceding claims,
**characterized in that**
the scan sequence step comprises
- at least one scanning for gathering ultrasound data of at least one region of interest (ROI) based on an activation of a sequence of sub-arrays (4) according to a rank-order of the sub-arrays (4).

6. The ultrasound system (1) according to claim 5,
**characterized in that**
the rank-order of the sub-arrays (4) has a rank-order limitation.

7. The ultrasound system (1) according to claim 5 or 6,
**characterized in that**
the scan sequence step comprises
determining the rank-order of the sub-arrays (4) based on a coverage information and a regularity information achievable by the sub-arrays (4), when operated.

8. The ultrasound system (1) according to claim 7,
**characterized in that**
the coverage information and the regularity information are obtainable by at least one initial scout scanning for gathering ultrasound data and/or by predetermined topography and/or location information.

9. The ultrasound system (1) according to one of the preceding claims,
**characterized in that**
the system (1) further comprises
- at least one reference module (43) configured for obtaining reference ultrasound data,
wherein the reference ultrasound data is obtained from the ultrasound data gathered by the scan sequence module (42).

10. The ultrasound system (1) according to one of the preceding claims,
**characterized in that**
the system (1) further comprises
- at least one feature detection module (44) configured for performing a determination step of the ultrasound data gathered by the scan sequence module (42) to detect at least one feature of interest (FOI).

11. The ultrasound system (1) according to claim 10,
**characterized in that**
the feature detection module (44) is configured to interface with the reference module (43) and to provide at least one detection information to the reference module (43) determined by the determination step.

12. Method for ultrasound imaging of a body part of a subject comprising at least the following steps:
- placing a transducer array (3) with a plurality of transducer array elements (3a) for providing ultrasound waves for transmission into the body of the subject, and
- performing a scan sequence step for obtaining ultrasound data based on reception of the ultrasound waves transmitted into the body of the subject.

13. The method according to claim 12,
**characterized in that**
the method is performed with an ultrasound system (1) according to one of claims 1 to 11.

14. The method according to claim 13,
**characterized in that**
placing further comprises
- aligning the transducer array (3) based on at least one landmark of the body of the subject, wherein, optionally, the landmark is a rib and/or the sternum of the subject.

15. The method according to one of claims 12 to 14,
**characterized in that**
the method further comprises
- performing a determination step of the obtained ultrasound data to detect at least one feature of interest (FOI), and
- obtaining reference ultrasound data based on at least one detection information determined by the determination step.
